(19) Europäisches Patentamt
European Patent Office
Office européen des brevets

(11) Veröffentlichungsnummer : **0 367 036 B1**

(12) # EUROPÄISCHE PATENTSCHRIFT

(45) Veröffentlichungstag der Patentschrift : **29.04.92 Patentblatt 92/18**

(51) Int. Cl.$^5$ : **C07D 271/04,** A61K 31/41

(21) Anmeldenummer : **89119456.5**

(22) Anmeldetag : **20.10.89**

(54) **Substituierte 3-Aminosydnonimine, Verfahren zu ihrer Herstellung und ihre Verwendung.**

(30) Priorität : **03.11.88 DE 3837327**

(43) Veröffentlichungstag der Anmeldung : **09.05.90 Patentblatt 90/19**

(45) Bekanntmachung des Hinweises auf die Patenterteilung : **29.04.92 Patentblatt 92/18**

(84) Benannte Vertragsstaaten : **AT BE CH DE ES FR GB GR IT LI NL SE**

(56) Entgegenhaltungen :
**EP-A- 0 346 684**
**US-A- 3 312 690**
**US-A- 3 769 283**

(73) Patentinhaber : **CASSELLA Aktiengesellschaft**
**Hanauer Landstrasse 526**
**W-6000 Frankfurt am Main 61 (DE)**

(72) Erfinder : **Schönafinger, Karl, Dr.**
**Holunderweg 8**
**W-8755 Alzenau (DE)**
Erfinder : **Beyerle, Rudi, Dr.**
**Mitterfeld 5d**
**W-8132 Tutzing (DE)**
Erfinder : **Just, Melitta, Dr.**
**Weimarer Strasse 2**
**W-6369 Nidderau 2 (DE)**
Erfinder : **Bohn, Helmut, Dr.**
**Kranzbergring 11**
**W-6369 Schöneck 1 (DE)**
Erfinder : **Ostrowski, Jörg, Dr.**
**Eschenstrasse 20**
**W-6458 Rodenbach 1 (DE)**

(74) Vertreter : **Urbach, Hans-Georg, Dr. et al**
**Hanauer Landstrasse 526**
**W-6000 Frankfurt am Main 61 (DE)**

EP 0 367 036 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft u.a. pharmakologisch wirksame substituierte 3-Aminosydnonimine.

Die US-A-3312690 offenbart Sydnonimine, die in 3-Stellung einen disubstituierten Aminorest tragen und die blutdrucksenkende Wirkung besitzen. Diese Sydnonimine besitzen in 5-Stellung eine freie Iminogruppe.

Die U.S-A-3769283 offenbart Sydnonimine, die in 3-Stellung einen disubstituierten Aminorest tragen und blutdrucksenkende Wirkung besitzen. Die Iminogruppe in 5-Stellung ist mit -NO oder z.B. mit Alkylcarbonyl substituiert.

Die EP-A-0346684, die unter Artikel 54 (3) EPÜ fällt, betrifft substituierte 3-Aminosydnomime mit Wirkung auf das Herz-Kreislaufsystem, bei denen die in 3-Stellung stehende Aminogruppe durch einen tert.-Butylrest und den Rest A-$(CH_2)_n$- disubstituiert ist, wobei n die Zahl 2 oder 3 und A z.B. Hydroxy, Alkoxy mit 1 bis 4 C-Atomen oder Dialkylamino mit insgesamt 2 bis 8 C-Atomen bedeutet.

Der Stand der Technik enthält keinerlei Hinweise darauf, daß Sydnoniminderivate mit einem monosubstituierten Aminorest in 3-Stellung pharmakologische Eigenschaften besitzen oder daß aus Sydnoniminen, die in 3-Stellung einen disubstituierten Aminorest besitzen, bei dem ein Substituent ein tert.-Butylrest ist, der tert.-Butylrest abgespalten werden kann.

Die Erfindung betrifft nun pharmakologisch wirksame substituierte 3-Aminosydnonimine der allgemeinen Formel I

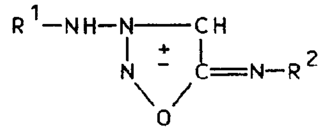

(I)

und ihre pharmakologisch annehmbaren .Säureadditionssalze, worin

$R^1$ Alkyl mit 1 bis 8 C-Atomen, Cycloalkyl mit 5 bis 8 C-Atomen, wobei der Alkyl- oder Cycloalkylrest auch mit $-OR^4$ oder $-N(R^5, R^6)$ substituiert sein kann, oder Adamantyl,

$R^2$ Wasserstoff, -NO oder den Rest $-COR^3$,

$R^3$ einen aliphatischen Rest mit 1 bis 6 C-Atomen, der auch mit Alkoxy mit 1 bis 6 C-Atomen oder mit einem Alkylthiorest mit 1 bis 4 C-Atomen oder mit Allylthio substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch mit Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen mit 1 bis 3 Halogenatomen und/oder 1 bis 3 Alkylresten mit 1 bis 3 C-Atomen und/oder 1 bis 3 Alkoxyresten mit 1 bis 3 C-Atomen und/oder 1 oder 2 Nitrogruppen und/oder 1 oder 2 Hydroxygruppen und/oder 1 oder 2 Alkylcarbonyloxyresten mit 1 bis 4 C-Atomen im Alkylteil mono-, di- oder trisubstituierten Arylrest mit 6 bis 10 C-Atomen; Imidazolyl, $R^4$, $R^5$, $R^6$ Wasserstoff oder Alkyl mit 1 bis 6 C-Atomen bedeuten.

Die Erfindung betrifft ferner ein Verfahren zur Herstellung der erfindungsgemäßen Verbindungen und ihre Verwendung, sowie einen Teil der zur Herstellung der erfindungsgemäßen Verbindungen benötigten Ausgangsprodukte.

Aliphatische Reste, Alkylreste, Alkoxyreste, Alkylaminoreste und Dialkylaminoreste können geradkettig oder verzweigt sein. Dies gilt auch, wenn sie als Substituenten anderer Reste auftreten.

Die für $R^5$ und $R^6$ stehenden Alkylreste können gleich oder verschieden sein.

Beispiele für $R^1$ sind: Methyl, Ethyl, n-Propyl, i-Propyl, n-, i-, sec.- und tert.-Butyl, n-, i-Pentyl, n-, i-Hexyl, n-, i-Heptyl, n-, i-Octyl, Cyclopentyl, Cyclohexyl, Cycloheptyl, Cyclooctyl, 2-Hydroxyethyl, 2- oder 3-Hydroxypropyl, 2-, 3- oder 4-Hydroxybutyl, 3-, 4- oder 5-Hydroxy-2-methyl-pentyl, 2-Methoxy-, -Ethoxy-, -Propoxy-ethyl, 3-Methoxy-, -Ethoxy-, -Propoxy-propyl, 3-Isopropoxy-propyl, 4-Butoxy-butyl, 2-Amino-, -Dimethylamino-, -Diethylamino-, -Methylethyl-amino-, -Methylpropylamino-ethyl, 4-Dibutylamino-butyl, 2- oder 3-Hydroxycyclopentyl, 2-, 3- oder 4-Hydroxycyclohexyl, 2-, 3- oder 4-Hydroxycycloheptyl, 2-, 3-, 4- oder 5-Hydroxycyclooctyl, 2-, .3- oder 4-Methoxy-, -Ethoxy-, -Propoxy-, -Butoxy-cyclohexyl, 2-, 3- oder 4-Amino-, -Dimethylamino-, -Diethylamino-, -Methylethylamino-cyclohexyl, 1-Adamantyl.

Für $R^1$ sind bevorzugt: Alkyl mit 1 bis 6 C-Atomen, ferner Hydroxyalkyl mit 2 bis 6 C-Atomen, Cyclohexyl und Hydroxycyclohexyl, insbesondere 2-Hydroxycyclohexyl, Adamantyl.

Besonders bevorzugte Reste für $R^1$ sind: Methyl, Ethyl, i-Propyl, Neopentyl, Cyclohexyl, 2-Hydroxyethyl, Adamantyl, 2-Hydroxycyclohexyl, von denen Ethyl, i-Propyl, Cyclohexyl, 2-Hydroxycyclohexyl und 1-Adamantyl ganz besonders bevorzugt sind.

Für $R^2$ sind bevorzugt: Wasserstoff, Acetyl, Propionyl, i-Propylcarbonyl, tert.-Butylcarbonyl, Cyclohexylcarbonyl, Benzoyl, 4-Chlorhenzoyl, Methoxycarbonyl, Ethoxycarbonyl, i-Propoxycarbonyl, Butoxycarbonyl, Allylthiomethylcarbonyl.

Als für $R^3$ stehende aliphatisrhe Reste kommen insbesondere Alkylreste, vorzugsweise mit 1 bis 4 C-Atomen, in Betracht. Die für $R^3$ stehenden aliphatischen Reste, insbesondere Alkylreste, können auch mit Alkoxy mit 1 bis 6 C-Atomen, insbesondere 1 bis 4 C-Atomen, vorzugsweise 1 bis 3 C-Atomen, substituiert sein. Beispiele für Alkyl- und Alkoxyalkylreste, die für $R^3$ stehen können, sind: Methyl; Ethyl; n-Propyl; i-Propyl; n-, i-, sec.- und tert.-Butyl; n- und i-Pentyl; n- und i-Hexyl; Methoxy-, Ethoxy-, n-Propoxy-, i-Propoxy-, n-Butoxy-, i-Butoxy- methyl; 2-Methoxy-, 2-Ethoxy-, 2-n-Propoxy-, 2-i-Propoxy-, 2-n-Butoxy-ethyl; 2-Methoxy-, 3-Ethoxy-, 3-n-Propoxy-, 3-i-Propoxy-propyl oder -i-propyl. Die für $R^3$ stehenden aliphastischen Reste, insbesondere die Alkylreste, können auch mit einem Alkylthiorest mit 1 bis 4 C-Atomen oder vorzugsweise mit Allylthio ($CH_2$=CH-$CH_2$-S) substituiert sein. Alkylthioreste mit 1 bis 4 C-Atomen sind zum Beipiel Methyl-, Ethyl-, n-Propyl-, i-Propyl-, n-Butyl-, tert.-Butyl-thio. Als für $R^3$ stehende cycloaliphatische Reste kommen vor allem Cycloalkylreste mit 5 bis 7 C-Atomen, insbesondere Cyclopentyl, und bevorzugt Cyclohexyl, in Betracht. Als für $R^3$ stehender bicycloaliphatischer Rest kommt insbesondere das 2,6,6-Trimethylbicyclo(3.1.1)heptan-3-yl (=Pinanyl-3) in Betracht. Als für $R^3$ stehender tricycloaliphatischer Rest kommt insbesondere das Tricyclo(3.3.1.1$^{3,7}$)decan-1-yl (=Adamantyl) in betracht.

Die für $R^3$ stehenden Alkoxyreste besitzen insbesondere 1 bis 4 C-Atome, vorzugsweise 1 oder 2 C-Atome. Die Alkoxysubstituenten für die Alkoxyreste besitzen insbesondere 1 bis 4 C-Atome. Beispiele für Alkoxyreste und Alkoxyalkoxyreste, die für $R^3$ stehen können sind: Methoxy; Ethoxy; n- und i-Propoxy; n-, i-, sec.- und tert.-Butoxy; n-Pentoxy; i-Hexoxy; Methoxy-, Ethoxy-, n-Propoxy-, i-Propoxy-, n-Butoxy-methoxy; 2-Methoxy-, 2-Ethoxy-, 2-n-Propoxy-, 2-i-Propoxy-ethoxy; 3-Methoxy-, 3-Ethoxy-, 3-n-Propoxy-, 3-i-Propoxy-propoxy; 4-Methoxy-, 4-Ethoxy-, 4-n-Propoxy-, 3-Propoxy-, 4-n-Butoxy-butoxy.

Der für $R^3$ stehende Alkoxycarbonylrest besitzt vorzugsweise 2 bis 5 C-Atome. Beispielsweise seien hierfür genannt.: Methoxy-, Ethoxy-, n-Propoxy-, i-Propoxy-, n-Butoxy-, i-Butoxy-carbonyl. Als für $R^3$ stehender Alkoxycarbonylrest kommt insbesondere der Ethoxycarbonylrest in Betracht.

Als für $R^3$ stehende Arylreste sind z.B. $\alpha$- oder $\beta$-Naphthylreste, insbesondere aber der Phenylrest, zu nennen. Als für $R^3$ stehende Aryloxyreste sind z.B. $\alpha$-oder $\beta$-Naphthoxyreste, insbesondere aber der Phenoxyrest, zu nennen. Die für $R^3$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Als Halogensubstituenten für die Arylreste kommen z.B. Fluor-, Chlor- und/oder Bromatome in Betracht. Als Alkylcarbonyloxysubstituenten für die Arylreste, insbesondere für einen Phenylrest, sind z.B. zu nennen: Acetoxy, n-Propionyloxy, i-Propionyloxy, n-Butyryloxy, i-Butyryloxy.

Beispiele für die für $R^3$ stehenden, gegebenenfalls substituierten Arylreste sind: Phenyl, 2-, ,3- oder 4-Methyl-, -Ethyl-, -n-Propyl-, -i-Propyl-phenyl; 2-, 3-oder 4-Methoxy-, -Ethoxy-, -n-Propoxy-, -i-Propoxyphenyl; 2-, 3- oder 4-Fluor-, -Chlor- oder -Brom-phenyl; 2-, 3- oder 4-Nitrophenyl; 2-, 3- oder 4-Hydroxyphenyl; 2-, 3- oder 4-Acetoxy-, -n-Propionyloxy-, -n-Butyryloxyphenyl; 2,3-, 2,4-, 2,5- oder 2,6-Dimethyl-, -Diethyl-, -Dipropyl-phenyl; 2- oder 3-Methyl-4-chlorphenyl; 2- oder 3-Ethyl-4-fluorphenyl; 2-Chlor-4-ethylphenyl; 2-Brom-4-i-propylphenyl; 2,6-Diethoxy-4-chlorphenyl; 2,3,4-, 3,4,5- oder 2,3,5-Trimethyl-, -Triethyl-, -Tripropyl-, -Trimethoxy-, -Triethoxy- oder Tripropoxy-phenyl; 2-Hydroxy-3-, -4- oder -5-chlorphenyl; 2-Methyl-3-, -4- oder -5-acetoxyphenyl.

Als für $R^3$ stehende substituierte Arylreste sind insbesondere zu nennen: Methylphenyl (= Tolyl), Nitrophenyl und Chlorphenyl. Der für $R^3$ stehende Imidazolylrest ist vorzugsweise ein 1-Imidazolylrest.

Rür $R^3$ sind bevorzugt: Methyl, Ethyl, Cyclohexyl, Phenyl, 4-Chlorphenyl, 4-Nitrophenyl, Methoxy, Ethoxy, n-Propoxy, i-Propoxy, 2-n-Propoxy-ethoxy, 2-i-Propoxy-ethoxy, n-Butoxymethyl, 2-n-Butoxy-ethoxy und Allylthiomethyl.

$R^4$ bedeutet vorzugsweise Wasserstoff. Die für $R^4$, $R^5$ und $R^6$ stehenden Alkylreste besitzen vorzugsweise 1 bis 4 C-Atome.

Eine Verbindung der Formel I kann dadurch hergestellt werden, daß

a) eine Verbindung der allgemeinen Formel II

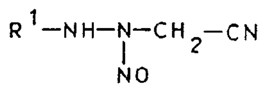

(II)

worin $R^1$ die bereits genannte Bedeutung besitzt, zu einer Verbindung der allgemeinen Formel Ia

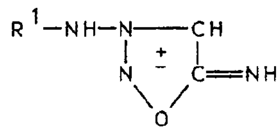

(Ia)

cyclisiert wird und daß diese oder ein Säureadditionssalz davon für den Fall, daß eine Verbindung der Formel I mit $R^2$ = $-COR^3$ hergestellt werden soll, mit einem Acylierungsmittel, das den Rest $-COR^3$ einführt, acyliert wird oder für den Fall, daß eine Verbindung der Formel I mit $R^2$ = -NO hergestellt werden soll, nitrosiert wird und die so erhaltene Verbindung gegebenenfalls in ein pharmakologisch annehmbares Säureadditionssalz überführt wird, oder daß

b) aus einem 3-Aminosydnonimin der Formel III

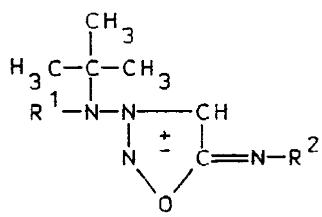

(III)

worin $R^1$ und $R^2$ die bereits genannten Bedeutungen besitzen, das auch in Form eines Säureadditionssalzes vorliegen kann, der tert.-Butylrest abgespalten und durch Wasserstoff ersetzt wird, wobei normalerweise zunächst eine Verbindung der Formel Ia entsteht, die - für den Fall, daß eine Verbindung der Formel I mit $R^2$ = $-COR^3$ hergestellt werden soll, wie vorstehend unter a) angegeben, direkt oder in Form eines Säureadditionssalzes mit einem Acylierungsmittel, das den Rest $-COR^3$ einführt, acyliert wird oder für den Fall, daß eine Verbindung der Formel I mit $R^2$ = -NO hergestellt werden soll, nitrosiert wird und die so erhaltene Verbindung gegebenenfalls in ein pharmakologisch annehmbares Säureadditionssalz überführt wird.

Die Herstellung der erfindungsgemäßen Verbindungen der Formel I nach der Verfahrensvariante b) ist bevorzugt.

Die Cyclisierung der Verbindung II zu der Verbindung Ia wird in einem geeigneten organischen oder anorganischan Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert von 3 oder darunter einstellen, also z.B. starke Säuren, wie Mineralsäuren, wie Schwefel-, Salpeter- oder Phosphorsäure, vorzugsweise Chlorwasserstoff, aber auch starke organische Säuren, wie Trifluoressigsäure. Die Cyclisierung wird normalerweise unter Eiskühlung durchgeführt. Von dem Cyclisierungsmittel kommt z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec.-, tert.-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Dibutyl-ether, Tetrahydrofuran, 1,4-Dioxan, 1,2-Dimethoxyethan, Bis-β-methoxyethylether; Oligoethylen-glykol-dimethyl-ether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl- oder -isobutylester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butyl-ester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig-und hochsiedende Petrolether, Spezialbenzine und Testbenzin; cycloaliphatische Kohlenwasserstoffe, wie z.B. Cyclopentan, Cyclohexan, Methylcyclohexan, Tetralin,

Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugsweise Essigsäureethylester-Methanol.

Die Verbindungen der Formel Ia stellen erfindungsgemäße Verbindungen der allgemeinen Formel I für den Fall dar, daß $R^2$ = Wasserstoff ist.

Die Acylierung der Verbindung der Formel Ia, die auch in Form eines Säureadditionssalzes vorliegen kann, zur Einführung des Restes $R^2 = -COR^3$ kann in an sich bekannter Weise mit einem geeigneten Acylierungsmittel der Formel IV durchgeführt werden

$$X-\overset{\overset{\displaystyle O}{\|}}{C}-R^3 \qquad (IV)$$

worin X ein nukleophil abspaltbarer Rest darstellt.

In der Formel IV bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyloder Nitrophenyl-Rest ist; $-O-CO-R^3$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird zweckmäßigerweise in flüssiger Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel IV theoretisch 1 : 1. Zweckmäßigerweise wird das Acylierungsmittel der Formel IV in einem geringen molaren Überschuß eingesetzt. Überschüsse von bis zu 30 mol% sind in der Regel ausreichend, d.h. das Molverhältnis zwischen der Verbindung der Formel Ia und dem Acylierungsmittel der Formel IV beträgt normalerweise 1 : (1 bis 1,3), vorzugsweise 1: (1 bis 1,2). Falls bei der Acylierungsreaktion eine Säure abgespalten wird, ist der Zusatz eines Säurefängers, wie z.B. eines Alkylihydroxids, wie z.B. Natrium-, Kalium- oder Lithium-hydroxid, eines tertiären organischen Amins, wie z.B. Pyridin oder Triethylamin, eines Alkalicarbonats oder Alkalibicarbonats, wie z.B. Soda oder Natriumbicarbonat., oder eines Alkalisalzes einer schwachen organischen Säure, wie z.B. Natriumacetat., zweckmäßig. Bei der Acylierungsreaktion können auch geeignete Katalysatoren, wie z.B. 4-Dimethylaminopyridin, zugesetzt werden.

Die Acylierung kann prinzipiell bei Temperaturen zwischen -10°C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel IV sind Acylierungsmittel und stellen somit z.B. dar: für X = Halogen Säurehalogenide bzw. Halogenameisensäureester, von denen Säurechloride und Chlorameisensäureester bevorzugt sind; für -OH Carbonsäuren; für -O-Alkyl und -O-Aryl Ester, von denen die Tolyl-, 2,4-Dinitro- oder 4-Nitrophenylester bevorzugt sind; für $-O-CO-R^3$ Anhydride; für -O-CO-O-Alkyl gemischte Carbonsäure-Kohlensäure-Anhydride; oder heterocyclische Amide oder Azolide, insbesondere von N,N′-Carbonyldiazolen, wie z.B. N,N′-Carbonyldiimidazol, 2,2′-Carbonyl-ditriazol(1,2,3), 1,1′-Carbonyl-ditriazol(1,2,4), N,N′-Carbonyldipyrazol, 2,2′-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel IV können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel IV zu überführen. Als derartige Aktivierungsmittel sind z.B. geeignet: N,N′-disubstituierte Carbodiimide, insbesondere wenn sie mindestens einen sekundären oder tertiären Alkylrest enthalten, wie z.B. Diisopropyl-, Dicyclohexyl- oder N-Methyl-N′-tert.butyl-carbodiimid (vgl. Methodicum Chimicum, Verlag G.Thieme, Stuttgart, Bd. 6, (1974), S.682/683, und Houben-Weyl, Methoden der Org.

Chemie, Bd. 8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N′-Disuccinimido-carbonat, Diphthalimido-carbonat, 1,1′-(Carbonyldioxy)-dibenzo-triazol oder Di-2-pyridyl-carbonat (vgl. z.B. Tetrahedron Letters, Vol.25, No. 43, 4943-4946), gegebenenfalls in Anwesenheit geeigneter Katalysatoren, wie z.B. 4-Dimethylaminopyridin. Ferner sind als Aktivierungsmittel N,N′-Carbonyldiazole, wie z.B. N,N′-Carbonyl-diimidazol, 2,2′-Carbonyl-ditriazol(1,2,3), 1,1′-Carbonyl-ditriazol(1,2,4), N,N′-Carbonyl-dipyrazol, 2,2′-Carbonyl-ditetrazol, N,N′-Carbonyl-benzimidazol oder N,N′-Carbonylbenztriazol, geeignet (vgl. z.B. H.A. Staab, M. Lücking und F.H. Dürr, loc. cit; H.A. Staab und A. Mannschreck loc. cit.; H.A. Staab und W. Rohr loc. cit). Als N,N′-Carbonyl-diazol wird häufig das käufliche N,N′-Carbonyl-diimidazol verwendet. Die anderen N,N′-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-PS 2 139 225) oder N,N′-Oxalyl-diazole wie z.B. 1,1′-Oxalyldi-imidazol, 1,1′-Oxalyldi-1,2,4-triazol und 1,1′-Oxalyldi-1,2,3,4-tetrazol (vgl. z.B. Shizuaka Murata, Bull.Chem. Soc.Jap. 57, 3597-3598 (1984)); Methylethylphosphinsäureanhydrid (vgl. z.B. DE-OS 31 01 427); Diphosphortetrajodid (Chem. Lett. 1983, 449); Dialkyldisulfit (Indian J. Chem. 21, 259 (1982)); oder andere reaktive Agentien.

Geeignete Lösungs-, Dispergier- oder Verdünnungsmittel für die Acylierung sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Falls eine Verbindung der Formel I mit $R^2$ = -NO hergestellt werden soll, wird eine Verbindung der Formel Ia, die auch in Form eines Säureadditionssalzes vorliegen kann, in an sich bekannter Weise, zweckmäßigerweise in einem geeigneten inerten Lösungsmittel oder Lösungsmittelgemisch, vorzugsweise in Wasser, normalerweise bei Temperaturen von 0 bis 40°C, und vorzugsweise bei Temperaturen von 0 bis 10°C, nitrosiert. Die Nitrosierung erfolgt mit salpetriger Säure, NO, NOCl oder NO-haltigen Gasgemischen. Zweckmäßigerweise erfolgt die Nitrosierung mit salpetriger Säure, die vorteilhafterweise aus einem Alkalimetallnitrit, z.B. Natriumnitrit, und einer Säure, insbesondere Salzsäure, erzeugt wird. Es ist zweckmäßig, die wäßrige Lösung der Verbindung Ia mit einer Säure, insbesondere Salzsäure, auf einen pH-Wert von 1 bis 3 einzustellen und das Alkalimetallnitrit in Form einer wäßrigen Lösung zu der gerührten und abgekühlten Lösung der Verbindung zuzutropfen.

Zur Herstellung einer erfindungsgemäßen Verbindung nach der Verfahrensvariante b) wird ein 3-Aminosydnonimin der Formel III, das auch in Form eines Säureadditionssalzes vorliegen kann, mit einer Säure behandelt. Für diese Behandlung genügen bereits katalytische Mengen an Säure. Es kann jedoch auch ein größerer molarer Säureüberschuß eingesetzt werden. Normalerweise wird das 3-Aminosydnonimin der Formel III oder ein Säureadditionssalz davon mit einer Säure im Molverhältnis 1 : (0,1 bis 5), vorzugsweise 1 : (0,1 bis ,0,5) in Kontakt gebracht. Diese Behandlung erfolgt zweckmäßigerweise ebenfalls in einem geeigneten organischen oder anorganischen Lösungs-, Dispergier- oder Verdünnungsmittel. Die Reaktionstemperatur beträgt normalerweise 0° bis zur Siedetemperatur des Reaktionsgemisches, insbesondere 0 bis 40°C, vorzugsweise 0 bis 20°C. Zur Abspaltung der tert.-Butylgruppe kann man eine Verbindung der Formel III bei Raumtemperatur längere Zeit mit einer Säure in einem geeigneten Lösungsmittel stehen lassen.

Als Lösungs-, Dispergier- oder Verdünnungsmittel kommen für die Verfahrensvariante b) z.B. diejenigen in Betracht, die für die Cyclisierung nach der Verfahrens variante a) angegeben worden sind.

Als Säuren für die Abspaltung der tert.-Butylgruppe können z.B. die bei der Verfahrensvariante a) als Cyclisierungsmittel genannten Säuren verwendet werden.

Bei der Verfahrensvariante b) fallen Säureadditionssalze der Verbindungen der Formel Ia an. Die Verbindungen der Formel Ia lassen sich durch die bereits genannte Acylierung oder Nitrosierung in anderer Verbindungen der Formel I überführen.

Die substituierten 3-Amino-sydnonimine der allgemeinen Formel I bilden mit anorganischen oder organischen Säuren Säureadditionssalze. Zur Bildung derartiger Säureadditionssalze sind anorganische oder organische Säuren geeignet. Geeignete Säuren sind beispielsweise Chlorwasserstoff, Bromwasserstoff, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen-, Adipinsäure, Naphthalindisulfonsäuren, insbesondere Naphthalindisulfonsäure(1,5). Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden.

Bei der Synthese der Verbindungen der Formel Ia fallen normalerweise die Säureadditionssalze an.

Die für den Syntheseweg a) benötigten Ausgangsverbindungen der allgemeinen Formel II können, sofern $R^1$ einen sperrigen Rest, wie z.B. eine sekundäre oder tertiäre Alkylgruppe oder Cycloalkyl, bedeutet, in an sich bekannter Weise nach der Strecker'schen Aminonitrilsynthese aus Hydrazinen der allgemeinen Formel V

$R^1$-NH-$NH_2$ (V)

worin $R^1$ die bereits genannte Bedeutung besitzt, durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel VI

$R^1$-NH-NH-$CH_2$-CN (VI)

entsteht, die durch Nitrosierung in die Verbindung II überführt wird. Die Nitrosierung wird in bekannter Weise in einem geeigneten inerten Lösungsmittel oder Lösungsmittelgemisch, vorzugsweise in Wasser, normalerweise bei Temperaturen von 0 bis 40°C, vorzugsweise bei Temperaturen von 0 bis 10°C durchgeführt. Die salpetrige Säure wird dabei normalerweise aus einem Alkalimetallnitrit, z.B. Natriumnitrit, und einer Säure, insbesondere Salzsäure, erzeugt. Es ist zweckmäßig, die wäßrige Lösung der Verbindung VI mit einer Säure, insbesondere Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Herstellung der Ausgangsverbindungen der Formel V ist bekannt oder kann nach an sich bekannten Verfahren durchgeführt werden.

Die für die Verfahrensvariante b) benötigten Ausgangsverbindungen der Formel III lassen sich ausgehend von Verbindungen der Formel VII

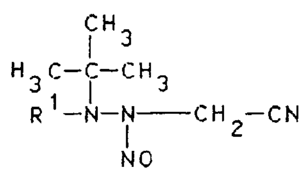

(VII)

durch Cyclisierung herstellen, wie dies bei der Verfahrensvariante a) beschrieben worden ist. Da die Cyclisierung durch Einwirkung von Säure durchgeführt wird, tritt bei längerer Säureeinwirkung nach erfolgter Cyclisierung eine Abspaltung der tert.-Butylgruppe ein. Das heißt, daß die Verfahrensvariante b) auch direkt anschließend an die Synthese der Verbindung III durchgeführt werden kann, ohne daß eine Isolierung der Verbindung III erfolgt.

Die benötigten Ausgangsverbindungen der allgemeinen Formel VII können in an sich bekannter Weise nach der Strecker'schen Aminonitrilsynthese aus Verbindungen der allgemeinen Formel VIII

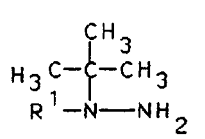

(VIII)

worin $R^1$ die bereits genannte Bedeutung besitzt, durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel IX

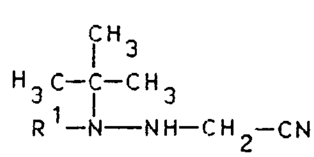

(IX)

entsteht, die durch Nitrosierung in die Verbindung VII überführt und anschließend cyclisiert wird, wie dies bereits im Zusammenhang mit den Verbindungen der Formel VI bei der Verfahrensvariante a) beschrieben worden ist.

Die Verbindungen der allgemeinen Formel VIII sind zum Teil bekannt bzw. lassen sich, ausgehend von Verbindungen der allgemeinen Formel X

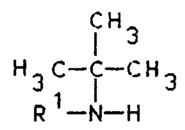

(X)

dadurch herstellen, daß entweder
eine Verbindung der Formel X zur N-Nitrosoverbindung XI nitrosiert und anschließend, zweckmäßigerweise mit Lithiumaluminiumhydrid, reduziert wird:

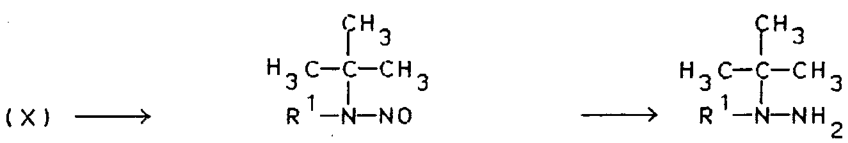

(XI) (VIII)

oder daß in an sich bekannter Weise eine Verbindung der Formel X mit Kaliumcyanat im sauren Medium in das Harnstoffderivat XII überführt wird, das dann durch Oxydation mit Natriumhypochlorit nach dem Hoffmann-Abbau in die Verbindung XIII überführt wird.

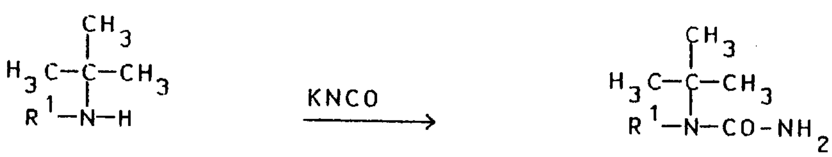


(X) (XII)

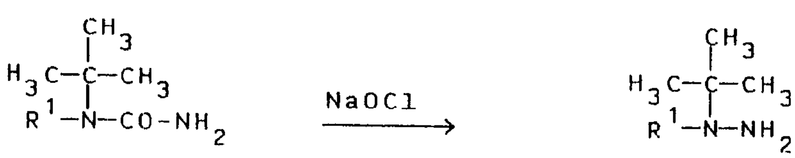


(XII) (VIII)

Die Herstellung der Ausgangsverbindungen der Formeln IV und X ist bekannt oder kann nach an sich bekannten Verfahren durchgeführt werden.

Die Verbindung der allgemeinen Formel I und ihre pharmakologisch annehmbaren Säureadditionssalze besitzen wertvolle pharmakologische Eigenschaften. Besonders ausgeprägt ist ihre Wirkung auf das Herz-Kreislauf-System. Verglichen mit bekannten, in 3-Stellung substituierten Sydnoniminverbindungen, z.B. solchen der EP-B-59356, sowie der im Handel befindlichen strukturähnlichen Verbindung Molsidomin, besitzen sie überraschenderweise eine wesentlich stärkere Wirkung und eine längere Wirkungsdauer. Die senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provizieren.

Durch Hemmung der Thrombocytenaggregation können die Verbindungen außerdem antithrombotische

Effekte zeigen.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine pharmakologisch wirksame Dosis mindestens einer Verbindung der Formel I oder eines Säureadditionssalzes davon, neben üblichen pharmazeutisch einwandfreien Träger- und Zusatzstoffen enthalten.

Die Heilmittel könzen oral, z.B in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saaccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacksoder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Benzafibrat, Fenofibrat; Mittel für die Thromboseprophylaxe, wie z.B. Phenprocoumon.

Die Verbindungen der Formel I, ihre pharmakologisch annehmbaren Säureadditionsssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

In den nachfolgenden Beispielen sind, sofern nichts anderes angegeben ist, Prozente in Gewichtsprozenten angegeben. Angegebene Verhältnisse zwischen Komponenten von Lösungs- oder Fließmitteln sind Volumenverhältnisse. Die Angabe "Zers." bedeutet "Zersetzung".

## Beispiel 1:

### a) 3-(2-Hydroxyethylamino)-sydnonimin-hydrochlorid

10 g 3-(tert.-Butyl-(2-hydroxyethyl)-amino)-sydnonimin-hydrochlorid werden in 100 ml 10%iger ethanolischer Salzsäure 14 Tage bei Raumtemperatur (20°C) aufbewahrt. Die Mischung wird 3 h im Eisbad gerührt und der Niederschlag abgesaugt und aus Ethanol umkristallisiert.

Ausbeute: 5,0 g Fp.: 145°C (Zers.)

Das als Ausgangsprodukt benötigte 3-(tert.-Butyl-(2-hydroxyethyl)-amino)-sydnonimin-hydrochlorid wird wie folgt hergestellt:

b) N-Nitroso-tert.-butyl-(2-hydroxyethyl)-amin

23,4 g 2-tert.-Butylamino-ethanol werden in 60 ml Wasser gelöst und mit 19,7 g konz. Salzsäure versetzt. Die Lösung von 20,7 g Natriumnitrit in 30 ml Wasser wird bei 30-40°C zugetropft. Nach beendeter Zugabe wird die Mischung Stunde auf 70°C erwärmt und dann im Eisbad gerührt. Das Produkt wird abgesaugt bzw. im Falle der öligen Derivate der nachfolgenden Beispiele mit Diethylether ausgeschüttelt und die Ether-Phase getrocknet und eingeengt.
Ausbeute: 23,9 g    Fp. 65-68°C

c) N-tert.-Butyl-N-(2-hydroxyethyl)-hydrazin

Die Mischung von 23 g N-Nitroso-tert.-butyl-(2-hydroxyethyl)-amin und 200 ml wasserfreies Tetrahydrofuran wird portionsweise bei 60°C mit insgesamt 12,2 g Lithiumalanat versetzt. Nach beendeter Zugabe wird noch 1 Stunde auf 60°C erwärmt, im Eisbad abgekühlt und das überschüssige Lithiumalanat durch vorsichtiges Zutropfen von Methanol und dann Wasser hydrolysiert. Die Feststoffe werden durch Absaugen entfernt, das Filtrat eingeengt. Es bleibt ein farbloses Öl (14 g) zurück, das ohne weitere Reinigung weiterverwendet wird.

d) 3-(tert.-Butyl-(2-hydroxyethyl)-amino)-sydnonimin-hydrochlorid

Das in der Stufe c erhaltene Öl (14 g) wird in 50 ml Wasser und 8,1 g konz. Salzsäure gelöst und auf -5°C abgekühlt. Dann wird die Lösung von 6,6 g Kaliumcyanid in 20 ml Wasser zugetropft. Nach Zugabe von 15 ml Ethanol wird eine 39%ige Formalinlösung (8,2 g) zugetropft und der pH-Wert der Mischung auf 7 eingestellt. Es wird 2 Stunden bei Raumtemperatur gerührt, mit konz. Salzsäure auf pH = 1,5 gestellt und unter Eiskühlung die Lösung von 5,6 g Natriumnitrit in 25 ml Wasser zugetropft. Es wird über Nacht bei Raumtemperatur nachgerührt und dann mit Diethylether extrahiert, getrocknet und eingeengt. Das zurückbleibende Öl wird in 50 ml konz. methanolische Salzsäure gelöst und mit 50 ml Essigsäureethylester verdünnt. Der ausgefallene Niederschlag wird abgesaugt und verworfen. Das Filtrat wird nach 2 Stunden eingeengt und der Rückstand mit Essigsäureethylester verrührt und der Feststoff abgesaugt und aus Ethanol/Diethylether (1:2) umkristallisiert.
Ausbeute: 5,9 g    Fp = 161-163°C (Zers.)

Beispiel 2:

3-Ethylamino-sydnonimin-hydrochlorid

Die Lösung von 26,55 g N-tert.-Butyl-N-ethyl-hydrazin-hydrochlorid und von 9,38 g Natriumcyanid in 100 ml Wasser wird bei 0 bis 5°C mit 14,5 g 39%igem Formalin versetzt. Diese Mischung wird 16 h bei Raumtemperatur gerührt, wobei der pH-Wert auf 7 bis 7,5 eingestellt wird. Durch Zugabe von konzentrierter Salzsäure wird dann auf pH 1,0 gestellt, die Mischung im Eisbad abgekühlt und eine Lösung von 9,5 g Natriumnitrit in 20 ml Wasser zugetropft. Die entstandene Nitrosoverbindung wird mit Ether extrahiert, getrocknet und eingeengt. Das verbleibende Öl wird mit 10 ml Isopropanol vermischt und mit in Essigester gelöstem Chlorwasserstoff versetzt und einen Tag lang bei Raumtemperatur gehalten. Die Bildung des Niederschlags wird durch Abkühlung auf 0°C verstärkt und der Niederschlag abgesaugt und aus Isopropanol umkristallisiert.
Ausbeute: 4,4 g    Fp.: 177°C (Zers.)

Beispiel 3:

N-Ethoxycarbonyl-3-ethylamino-sydnonimin

Die auf 0°C abgekühlte Lösung von 7,9 g 3-Ethylamino-sydnonimin-hydrochlorid in 30 ml Wasser wird mit 7,5 g Natriumbicarbonat und anschließend mit einer Lösung von 5,8 g Chlorameisensäureethylester in 30 ml Methylenchlorid versetzt und 15 h bei Raumtemperatur gerührt. Die Methylenchloridphase wird abgetrennt, getrocknet und eingeengt. Das verbleibende Öl wird säulenchromatographisch (Kieselgel; $CH_2Cl_2$:MeOH= 98:2) gereinigt und durch Verrühren mit Diisopropylether zur Kristallisation gebracht.
Ausbeute: 4,6 g    Fp.: 131°C (Zers.)

Beispiel 4:

3-Cyclohexylamino-sydnonimin-hydrochlorid

a) N-Nitroso-tert.-butyl-cyclohexyl-amin

Die Mischung bestehend aus 13,2 ml 10N Salzsäure, 50 ml Wasser, 18,8 g tert.-Butyl-cyclohexyl-amin und 17,9 g Natriummitrit wird 3 h auf 90°C erwärmt. Beim Abkühlen fällt ein Feststoff aus, der abgesaugt wird.
Ausbeute: 16,3 g Fp.: 87°C

b) N-tert.-Butyl-N-cyclohexyl-hydrazin-hydrochlorid

Die Mischung aus 50 ml Tetrahydrofuran, 100 ml Dibutylether, 0,38 g Lithiumalanat und 1,8 g Nitroso-tert.-butyl-cyclohexyl-amin wird 2 h unter Stickstoff zum Sieden erhitzt. Dann werden 14,5 g Nitrosoverbindung auf einmal und insgesamt 3,8 g Lithiumalanat in 3 Portionen innerhalb von 5 Stunden zugefügt und das Gemisch währenddessen auf 90°C erwärmt. Nach Beendigung der Umsetzung wird das restliche Lithiumalanat durch vorsichtiges Zutropfen von Wasser unter Kühlung vernichtet, der sich bildende Feststoff abgesaugt und das Filtrat mit 2 Portionen 1N Salzsäure (je 100 ml) ausgeschüttelt. Die wäßrige Phase, die das Hydrazinhydrochlorid enthält, wird mit Ether ausgeschüttelt und für die nächste Stufe ohne weitere Reinigung eingesetzt.

c) N-tert.-Butyl-N-cyclohexyl-N′-cyanmethyl-hydrazin

Die unter b erhaltene Lösung des Hydrazinhydrochlorids wird im Eisbad gerührt und mit 4 g Natriumcyanid versetzt. Die Mischung wird neutral gestellt, und 7,7 g einer 39%igen Formalinlösung werden zugetropft. Durch Zugabe von Sodalösung wird das pH auf einen Wert von 7 eingestellt, 2 Stunden bei Raumtemperatur gerührt und das Produkt mit diethylether extrahiert. Nach dem Trocknen und Einengen verbleibt ein gelbes Öl, das ohne weitere Reinigung für die nächste Stufe eingesetzt wird.
Ausbeute: 12,2 g

d) 3-(tert.-Butyl-cyclohexyl-amino)-sydnonimin-hydrochlorid

Das unter c erhaltene Öl wird in 100 ml Wasser gelöst und mit 6 ml 10N HCl versetzt. Dann wird die Lösung von 6,9 g Natriumnitrit in 20 ml Wasser zugetropft und eine Stunde bei Raumtemperatur nachgerührt. Die Nitrosozwischenstufe wird mit Essigester (50 ml) ausgeschüttelt, die Essigesterphase getrocknet und mit überschüssiger etherischer Salzsäure bei 0-5°C behandelt. Das sich abscheidende Öl wird säulenchromatographisch (Kieselgel, MeOH:$CH_2Cl_2$=1:9) gereinigt. Nach dem Einengen der entsprechenden Fraktionen im Rotationsverdampfer wird der Rückstand mit Diethylether verrührt und der Feststoff abgesaugt.
Ausbeute: 6,7 g Fp.: 158°C (Zers.)

e) 3-Cyclohexylamino-sydnonimin-hydrochlorid

Die Lösung von 4 g 3-(tert.-Butyl-N-cyclohexyl-amino)-sydnonimin-hydrochlorid in 30 ml konz. ethanolischer Salzsäure wird einen Tag bei Raumtemperatur stehen gelassen und dann im Rotationsverdampfer eingeengt. Der Rückstand wird säulenchromatografisch (Kieselgel, $CH_2Cl_2$: MeOH=9:1) gereinigt und der nach dem Einengen der entsprechenden Fraktionen erhaltene Rückstand mit Essigsäureisopropylester verrührt und abgesaugt.
Ausbeute: 2,5 g Fp.: 90°C (Zers.)

Beispiel 5

3-(2-Hydroxycylohexyl-amino)-sydnonimin-hydrochlorid

a) N-tert.-Butyl-N-(2-hydroxycylohexyl)-hydrazin-hydrochlorid

Zur Lösung von 38,8 g N-Nitroso-tert.-butyl-(2-hydroxycyclohexyl)-amin (durch Erhitzen von 1.2-Epoxycyclohexan mit tert.-Butylamin auf 150°C im Autoklaven und anschließendes Nitrosieren mit Natriumnitrit/HCl gewonnen) werden unter Stickstoffatmosphäre 10 g Lithiumalanat in Portionen von 1 g innerhalb von 2 Tagen zugegeben, wobei die Mischung auf 50°C erwärmt wird. Das restliche Lithiumalanat wird durch Abkühlen der

Mischung und vorsichtiges Zutropfen von 50 ml Methanol und anschließend 75 ml Wasser vernichtet. Die Mischung wird dann filtriert und das Filtrat eingeengt. Der Rückstand wird in 100 ml Methylenchlorid aufgenommen, das sich abscheidende Wasser abgetrennt und die organische Phase getrocknet und erneut eingeengt. Das so erhältliche Öl wird in 20 ml Ethanol und 150 ml tert.-Butylmethylether gelöst und das Hydrazinhydrochlorid durch Zugabe von etherischer HCl gefällt.
Ausbeute: 28,5 g Fp.: 145°C (Zers.)

b) 3-(2-Hydroxycylohexyl-amino)-sydnonimin-hydrochlorid

Die Mischung aus 13,35 g N-tert.-Butyl-N-(2-hydroxycylohexyl)-hydrazin-hydrochlorid, 3,1 g Natriumcyanid und 50 ml Wasser wird im Eisbad gerührt und tropfenweise mit einer 39%igen Formalinlösung (5,7 g) versetzt. Der pH-Wert der Mischung wird auf 7 bis 7,5 eingestellt und 3 Stunden bei Raumtemperatur gerührt. Dann wird mit konz. Salzsäure auf pH=1 gestellt und die Lösung von 6 g Natriumnitrit in 25 ml Wasser zugetropft. Nach einer Stunde wird die Nitrosoverbindung mit Diethylether extrahiert, die etherische Phase getrocknet und mit etherischer HCl versetzt. Nach 2 Tagen wird der Niederschlag abgesaugt und aus Isopropanol/Essigester umkristallisiert.
Ausbeute: 4,5 g Fp.: 174-5°C (Zers.)

Beispiel 6

N-Benzoyl-3-(2-hydroxycylohexyl-amino-sydnonimin

Die auf 5°C abgekühlte Lösung von 1,9 g 3-(2-Hydroxycylohexyl-amino)-sydnonimin-hydrochlorid und 1,7 g Natriumbicarbonat in 20 ml Wasser wird mit der Lösung von 1,45 g Benzoylchlorid in 20 ml Methylenchlorid versetzt und 4 Stunden im Eisbad gerührt. Der Niederschlag wird abgesaugt und aus Isopropanol umkristallisiert.
Ausbeute: 1,6 g Fp.: 174°C (Zers.)

Beispiel 7

N-Cyclohexylcarbonyl-3-(2-hydroxycylohexyl-amino)-sydnonim

Die auf 5°C abgekühlte Lösung von 3,5 g 3-(2-Hydroxycyclohexyl-amino)-sydnonimin-hydrochlorid und 2,6 g Natriumbicarbonat in 30 ml Wasser wird mit der Lösung von 3,3 g Cyclohexancarbonsäurechlorid in 30 ml Methylen chlorid versetzt und 20 Stunden bei Raumtemperatur gerührt. Die Methylenchloridphase wird abgetrennt, getrocknet und eingeengt. Der Rückstand wird aus Essigsäureisopropylester umkristallisiert.
Ausbeute: 2,6 g Fp.: 155°C (Zers.)

Beispiel 8

N-(4-Chlorbenzoyl-3-(2-hydroxycylohexyl-amino)-sydnonim

Die Lösung von 3,5 g 3-(2-Hydroxycylohexyl-amino)-sydnonim-hydrochlorid in 30 ml Wasser wird auf 5°C abgekühlt und nacheinandermit 2,6 g Natriumcarbonat und der Lösung von 3,9 g 4-Chlorbenzoylchlorid in 30 ml Methylenchlorid versetzt und 20 Stunden bei Raumtemperatur gerührt. Die organische Phase wird abgetrennt, getrocknet und eingeengt. Der ölige Rückstand wird aus Di-n-propylether kristallisiert.
Ausbeute: 3,3 g Fp.: 169°C (Zers.)

Beispiel 9

N-Ethoxycarbonyl-3-(2-hydroxycylohexyl-amino-sydnonim

Diese Verbindung wird analog Beispiel 8 aus 7,6 g 3-(2-Hydroxycylohexyl-amino)-sydnonim-hydrochlorid, 3,4 g Chlorameisensäureethylester und 5,5 g Natriumbicarbonat hergestellt und aus Essigsäureisopropylester umkristallisiert.
Ausbeute: 5,8 g Fp.: 147°C

Die nachfolgenden Beispiele A bis F betreffen pharmazeutische Präparate.

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | pro ml |
|---|---|
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

Emulsion, enthaltend 3 mg Wirkstoff pro 5 ml

| | pro 100 ml Emulsion |
|---|---|
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

Rektale Arzneiform, enthaltend 4 mg Wirkstoff pro Suppositorium

| | pro Suppositorium |
|---|---|
| Wirkstoff | 4 mg |
| Suppositoriengrundmasse | ad 2 g |

Beispiel E

Tabletten, enthaltend 2 mg Wirkstoff pro Tablette

| | pro Tablette |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel F

Dragees, enthaltend 1 mg Wirkstoff pro Dragee

| | pro Dragee |
|---|---|
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

Die pharmakologische Wirkung der Verbindung der Formel I wurde nach einer modifizierten Methode von Godfraind and Kaba (Arch. Int. Pharmacodyn. Ther. 196, (Suppl) 35 bis 49, 1972) und von Schümann et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Die relaxierende Wirkung der Prüfsubstanz wird durch kumulative Zugabe von in 1/2 log 10 ab gestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50% hemmt (= $IC_{50}$, mol/l). In der folgenden Tabelle sind die so erhaltenen $IC_{50}$-Werte angegeben. Wie der Vergleich mit dem $IC_{50}$-Werte:> $1 \cdot 10^{-4}$ für die bekannte Verbindung Molsidomin (N-Ethoxycarbonyl-3-morpholino-sydnonimin), vgl. DE-B-16 95 897, ergibt, liegen die Werte für die Verbindungen der Formel I erheblich günstiger.

Tabelle

| Verbindung der Formel I gemäß Beispiel | $IC_{50}$ (mol/l) |
|---|---|
| 1a | $2 \cdot 10^{-6}$ |
| 2 | $2 \cdot 10^{-6}$ |
| 4e | $2 \cdot 10^{-6}$ |
| 5b | $1 \cdot 10^{-6}$ |
| Molsidomin (Vergleichssubstanz) | $> 1 \cdot 10^{-4}$ |

**Patentansprüche**

**Patentansprüche für folgende Vertragsstaaten : DE, GB, FR, IT, NL, SE, LI, CH, BE, AT**

1. Pharmakologisch wirksame substituierte 3-Aminosydnonimine der allgemeinen Formel I

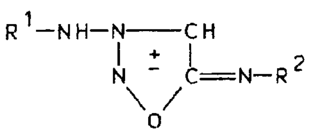

(I)

und ihre pharmakologisch annehmbaren Säureadditionssalze, worin

$R^1$ Alkyl mit 1 bis 8 C-Atomen, Cycloalkyl mit 5 bis 8 C-Atomen, wobei der Alkyl- oder Cycloalkylrest auch mit $-OR^4$ oder $-N(R^5, R^6)$ substituiert sein kann, oder Adamantyl,

$R^2$ Wasserstoff, -NO oder den Rest $-COR^3$,

$R^3$ einen aliphatischen Rest mit 1 bis 6 C-Atomen, der auch mit Alkoxy mit 1 bis 6 C-Atomen oder mit einem Alkylthiorest mit 1 bis zu 4 C-Atomen oder mit Allylthio substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch mit Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen mit 1 bis 3 Halogenatomen und/oder 1 bis 3 Alkylresten mit 1 bis 3 C-Atomen und/oder 1 bis 3 Alkoxyresten mit 1 bis 3 C-Atomen und/oder 1 oder 2 Nitrogruppen und/oder 1 oder 2 Hydroxygruppen und/oder 1 oder 2 Alkylcarbonyloxyresten mit 1 bis 4 C-Atomen im Alkylteil mono-, di- oder trisubstituierten Arylrest mit 6 bis 10 C-Atomen; Imidazolyl,

$R^4$, $R^5$, $R^6$ Wasserstoff oder Alkyl mit 1 bis 6 C-Atomen bedeuten.

2. Substituierte 3-Aminosydnonimine nach Anspruch 1, dadurch gekennzeichnet, daß $R^1$ Alkyl mit 1 bis 6 C-Atomen, Hydroxyalkyl mit 2 bis 6 C-Atomen, Cyclohexyl, Hydroxycyclohexyl oder Adamantyl bedeutet.

3. Substituierte 3-Aminosydnonimine nach Anspruch 1 und/oder 2, dadurch gekennzeichnet, daß $R^2$ Wasserstoff Acetyl, Propionyl, i-Propylcarbonyl, tert.-Butylcarbonyl, Cyclohexylcarbonyl, Benzoyl, 4-Chlorbenzoyl, Methoxycarbonyl, Ethoxycarbonyl, i-Propoxycarbonyl, Butoxycarbonyl oder Allylthiomethylcarbonyl bedeutet.

4. 3-Cyclohexylamino-sydnonimin und seine pharmakologisch annehmbaren Säureadditionssalze, insbesondere sein Hydrochlorid.

5. 3-(Adamant-1-yl)-amino-sydnonimin und seine pharmakologisch annehmbaren Säureadditionssalze, insbesondere sein Hydrochlorid.

6. 3-(2-Hydroxycyclohexyl)-amino-sydnonimin und seine pharmakologisch annehmbaren Säureadditionssalze, insbesondere sein Hydrochlorid.

7. Verfahren zur Herstellung der in einem oder mehreren der Ansprüche 1 bis 3 angegebenen Verbindungen der Formel I oder der in den Ansprüchen 4 bis 6 angegebenen Verbindungen, dadurch gekennzeichnet, daß

a) eine Verbindung der allgemeinen Formel II

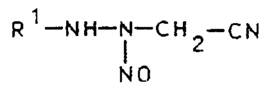

(II)

worin $R^1$ die bereits genannte Bedeutung besitzt, zu einer Verbindung der allgemeinen Formel Ia

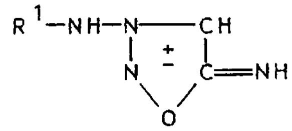

(Ia)

cyclisiert wird und daß diese oder ein Säureadditionssalz davon für den Fall, daß eine Verbindung der Formel I mit $R^2$ = -$COR^3$ hergestellt werden soll, mit einem Acylierungsmittel, das den Rest -$COR^3$ einführt, acyliert wird oder für den Fall, daß eine Verbindung der Formel I mit $R^2$ = -NO hergestellt werden soll, nitrosiert wird und die so erhaltene Verbindung gegebenenfalls in ein pharmakologisch annehmbares Säureadditionssalz überführt wird, oder daß

b) aus einem 3-Aminosydnonimin der Formel III

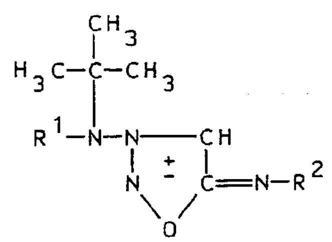

(III)

worin $R^1$ und $R^2$ die bereits genannten Bedeutungen besitzen, das auch in Form eines Säureadditionssalzes vorliegen kann, der tert-Butylrest abgespalten und durch Wasserstoff ersetzt wird, wobei zunächst eine Verbindung der Formel Ia entsteht und daß für den Fall, daß eine Verbindung der Formel I mit $R^2$ = $COR^3$ hergestellt wird, die Verbindung der Formel Ia oder ein Säureadditionssalze davon mit einem Acylierungsmittel acyliert wird, das den Rest -$COR^3$ einführt oder für den Fall, daß eine Verbindung der Formel I mit $R^2$ = -NO hergestellt werden soll, nitrosiert wird und die erhaltene Verbindung gegebenenfalls in ein pharmakologisch annehmbares Säureadditionssalz überführt wird.

8. Verfahren nach Anspruch 7, dadurch gekennzeichnet, daß die Cyclisierung oder Abspaltung des tert.-Butylrestes in einem Lösungs-, Dispergier- oder Verdünnungsmittel bei Temperaturen von 0 bis 40°C, vorzugsweise 0 bis 20°C, mit Hilfe einer Säure, die in wäßriger Lösung einen pH-Wert von 3 oder darunter einstellt, durchgeführt wird.

9. Substituierte 3-Aminosydnonimine der Ansprüche 1 bis 6 oder ihre pharmakologisch annehmbaren Säureadditionssalze als pharmakologische Wirkstoffe zur Anwendung bei der Bekämpfung und Vorbeugung cardiovaskulärer Erkrankungen.

10. Pharmazeutisches Präparat, dadurch gekennzeichnet, daß es eine Verbindung der Ansprüche 1 bis 6 oder ein pharmakologisch annehmbares Säureadditionssalz davon als Wirkstoff zusammen mit pharmazeutisch annehmbaren Träger- und Zusatzstoffen und gegebenenfalls noch eine oder mehrere andere pharmakologische Wirkstoffe enthält.

11. Substituierte 3-Aminosydnonimine der allgemeinen Formel III

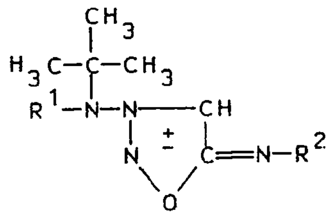

(III)

und ihre Säureadditionssalze, worin

$R^1$ Alkyl mit 1 bis 8 C-Atomen, Cycloalkyl mit 5 bis 8 C-Atomen, wobei der Cycloalkylrest oder ein Alkylrest mit 4 bis 8 C-Atomen auch mit $-OR^4$ oder $-N(R^5, R^6)$ substituiert sein kann, oder Adamantyl,

$R^2$ Wasserstoff, -NO oder den Rest $-COR^3$,

$R^3$ einen aliphatischen Rest mit 1 bis 6 C-Atomen, der auch mit Alkoxy mit 1 bis 6 C-Atomen oder mit einem Alkylthiorest mit 1 bis 4 C-Atomen oder mit Allylthio substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch mit Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen mit 1 bis 3 Halogenatomen und/oder 1 bis 3 Alkylresten mit 1 bis 3 C-Atomen und/oder 1 bis 3 Alkoxyresten mit 1 bis 3 C-Atomen und/oder 1 oder 2 Nitrogruppen und/oder 1 oder 2 Hydroxygruppen und/oder 1 oder 2 Alkylcarbonyloxyresten mit 1 bis 4 C-Atomen im Alkylteil mono-, di- oder trisubstituierten Arylrest mit 6 bis 10 C-Atomen; Imidazolyl, $R^4$, $R^5$, $R^6$ Wasserstoff oder Alkyl mit 1 bis 6 C-Atomen bedeuten.

**Patentansprüche für folgende Vertragsstaaten : GR, ES**

1. Verfahren zur Herstellung pharmakologisch wirksamer substituierter 3-Aminosydnonimine der allgemeinen Formel I

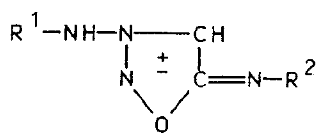

(I)

und ihrer pharmakologisch annehmbaren Säureadditionssalze, worin

$R^1$ Alkyl mit 1 bis 8 C-Atomen, Cycloalkyl mit 5 bis 8 C-Atomen, wobei der Alkyl- oder Cycloalkylrest auch mit $-OR^4$ oder $-N(R^5, R^6)$ substituiert sein kann, oder Adamantyl,

$R^2$ Wasserstoff, -NO oder den Rest $-COR^3$,

$R^3$ einen aliphatischen Rest mit 1 bis 6 C-Atomen, der auch mit Alkoxy mit 1 bis 6 C-Atomen oder mit einem Alkylthiorest mit 1 bis zu 4 C-Atomen oder mit Allythio substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch mit Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen mit 1 bis 3 Halogenatomen und/oder 1 bis 3 Alkylresten mit 1 bis 3 C-Atomen und/oder 1 bis 3 Alkoxyresten mit 1 bis 3 C-Atomen und/oder 1 oder 2 Nitrogruppen und/oder 1 oder 2 Hydroxygruppen und/oder 1 oder 2 Alkcarbonyloxyresten mit 1 bis 4 C-Atomen im Alkylteil mono-, di- oder trisubstituierten Arylrest mit 6 bis 10 C-Atomen; Imidazolyl,

$R^4$, $R^5$, $R^6$ Wasserstoff oder Alkyl mit 1 bis 6 C-Atomen bedeuten, dadurch gekennzeichnet, daß

a) eine Verbindung der allgemeinen Formel II

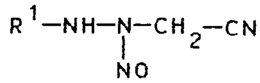

(II)

worin $R^1$ die bereits genannte Bedeutung besitzt, zu einer Verbindung der allgemeinen Formel Ia

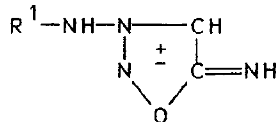

(Ia)

cyclisiert wird und daß diese oder ein Säureadditionssalz davon für den fall, daß eine Verbindung der Formel I mit $R^2$ = $-COR^3$ hergestellt werden soll, mit einem Acylierungsmittel, das den Rest $-COR^3$ einführt, acyliert wird oder für den fall, daß eine Verbindung der Formel I mit $R^2$ = -NO hergestellt. werden soll, nitrosiert wird und die so erhaltene Verbindung gegebenenfalls in ein pharmakologisch annehmbares Säureadditionssalz überführt wird, oder daß
b) aus einem 3-Aminosydnonimin der Formel III

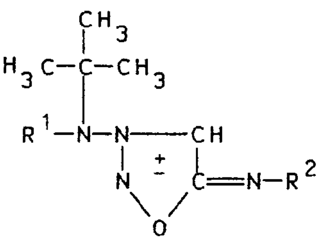

(III)

worin $R^1$ und $R^2$ die bereits genannten Bedeutungen besitzen, das auch in Form eines Säureadditionssalzes vorliegen kann, der tert-Butylrest abgespalten und durch Wasserstoff ersetzt wird, wobei zunächst eine Verbindung der Formel Ia entsteht und daß für den Fall, daß eine Verbindung der Formel I mit $R^2$ = $-COR^3$ hergestellt wird, die Verbindung der Formel Ia oder ein Säureadditionssalz davon mit einem Acylierungsmittel acyliert wird, das den Rest $-COR^3$ einführt oder für den Fall, daß eine Verbindung der Formel I mit $R^2$ = -NO hergestellt werden soll, nitrosiert wird und die erhaltene Verbindung gegebenenfalls in ein pharmakologisch annehmbares Säureadditionssalz überführt wird.

2. Verfahren nach Anspruch 1, dadurch gekennzeichnet, daß die Cyclisierung oder Abspaltung des tert.-Butylrestes in einem Lösungs-, Dispergier- oder Verdünnungsmittel bei Temperaturen von 0 bis 40°C, vorzugsweise 0 bis 20°C, mit Hilfe einer Säure, die in wäßriger Lösung einen pH-Wert von 3 oder darunter einstellt, durchgeführt wird.

3. Verfahren nach Anspruch 1 und/oder 2, dadurch gekennzeichnet, daß die Ausgangsprodukte so ausgewählt werden, daß eine Verbindung der Formel I entsteht, bei der $R^1$ Alkyl mit 1 bis 6 C-Atomen, Hydroxyalkyl mit 2 bis 6 C-Atomen, Cyclohexyl, Hydroxycyclohexyl oder Adamantyl bedeutet.

4. Verfahren nach einem oder mehreren der Ansprüche 1 bis 3, dadurch gekennzeichnet, daß die Ausgangsprodukte so ausgewählt werden, daß eine Verbindung der Formel I entsteht, bei der $R^2$ Wasserstoff Acetyl, Propionyl, i-Propylcarbonyl, tert.-Butylcarbonyl, Cyclohexylcarbonyl, Benzoyl, 4-Chlorbenzoyl, Methoxycarbonyl, Ethoxycarbonyl, i-Propoxycarbonyl, Butoxycarbonyl oder Allylthiomethylcarbonyl bedeutet.

5. Verfahren nach einem oder mehreren der Ansprüche 1 bis 4, dadurch gekennzeichnet, daß die Ausgangsprodukte so ausgewählt werden, daß die Verbindung 3-Cyclohexylamino-sydnonimin oder ein pharmakologisch annehmbares Säureadditionssalz davon, insbesondere ihr Hydrochlorid, entsteht.

6. Verfahren nach einem oder mehreren der Ansprüche 1 bis 4, dadurch gekennzeichnet, daß die Ausgangsprodukte so ausgewählt werden, daß die Verbindung 3-(Adamant-1-yl)-amino-sydnonimin oder ein pharmakologisch annehmbares Säureadditionssalz davon, insbesondere ihr Hydrochlorid, entsteht.

7. Verfahren nach einem oder mehreren der Ansprüche 1 bis 4, dadurch gekennzeichnet, daß die Ausgangsprodukte so ausgewählt werden, daß die Verbindung 3-(2-Hydroxycyclohexyl)-amino-sydnonimin oder ein pharmakologisch annehmbares Säureadditionssalz davon, insbesondere ihr Hydrochlorid, entsteht.

8. Anwendung des nach dem Verfahren eines oder mehrerer der Ansprüche 1 bis 7 hergestellten substituierten 3-Aminosydnonimins oder eines seiner pharmakologisch annehmbaren Säureadditionssalze als pharmakologische Wirkstoffe bei der Bekämpfung und Vorbeugung cardiovaskulärer Erkrankungen.

9. Verfahren zur Herstellung eines pharmazeutischen Präparates, dadurch gekennzeichnet, daß ein nach dem Verfahren eines oder mehrerer der Ansprüche 1 bis 7 hergestelltes substituiertes 3-Aminosydnonimin oder ein pharmakologisch annehmbares Säureadditionssalz davon als Wirkstoff zusammen mit pharmazeutisch annehmbaren Träger-und Zusatzstoffen und gegebenenfalls noch einen oder mehrere andere pharmakologische Wirkstoffe in an sich bekannter Weise in ein pharmazeutisches Präparat überführt wird.

10. Verfahren zur Herstellung substituierter 3-Aminosydnonimine der allgemeinen Formel III

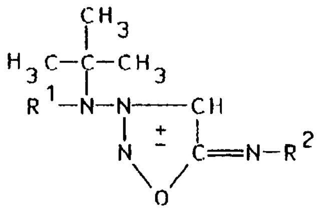

(III)

und ihrer Säureadditionssalze, worin

$R^1$ Alkyl mit 1 bis 8 C-Atomen, Cycloalkyl mit 5 bis 8 C-Atomen, wobei der Cycloalkylrest oder ein Alkylrest mit. 4 bis 8 C-Atomen auch mit -$OR^4$ oder -$N(R^5, R^6)$ substituiert. sein kann, oder Adamantyl,

$R^2$ Wasserstoff, -NO oder den Rest -$COR^3$,

$R^3$ einen aliphatischen Rest mit 1 bis 6 C-Atomen, der auch mit Alkoxy mit 1 bis 6 C-Atomen oder mit einem Alkylthiorest mit 1 bis 4 C-Atomen oder mit Allylthio substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen, der auch mit Alkoxy mit 1 bis 6 C-Atomen substituiert sein kann; einen Aryloxyrest mit 6 bis 10 C-Atomen; einen Alkoxycarbonylrest mit insgesamt 2 bis 7 C-Atomen; einen Arylrest. mit 6 bis 10 C-Atomen; einen mit 1 bis 3 Halogenatomen und/oder 1 bis 3 Alkylresten mit 1 bis 3 C-AtOmen und/oder 1 bis .3 Alkoxyresten mit 1 bis 3 C-AtOmen und/oder 1 oder 2 Nitrogruppen und/oder 1 oder 2 Hydroxygruppen und/oder 1 oder 2 Alkylcarbonyloxyresten mit 1 bis 4 C-Atomen im Alkylteil mono-, di- oder trisubstituierten Arylrest. mit 6 bis 10 C-Atomen; Imidazolyl, $R^4$, $R^5$, $R^6$ Wasserstoff oder Alkyl mit 1 bis 6 C-Atomen bedeuten, dadurch gekennzeichnet, daß eine Verbindung der allgemeinen Formel VII

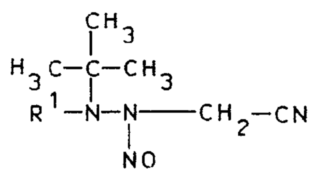

(VII)

cyclisiert wird.

## Claims

### Claims for the following Contracting States : AT, BE, CH, DE, FR, GB, IT, LI, NL, SE

1. Pharmacologically active substituted 3-aminosydnonimines of the general formula I

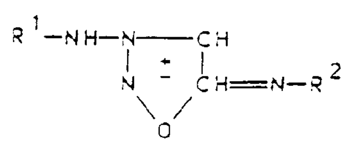

(I)

and their pharmacologically acceptable acid addition salts, wherein

$R^1$ denotes alkyl having 1 to 8 C atoms, cycloalkyl having 5 to 8 C atoms, it also being possible for the alkyl or cycloalkyl radical to be substituted by -$OR^4$ or -$N(R^5, R^6)$, or adamantyl,

$R^2$ denotes hydrogen, -NO or the radical -$COR^3$,

$R^3$ denotes an aliphatic radical having 1 to 6 C atoms, which can also be substituted by alkoxy having 1 to 6 C atoms or by an alkyl thio radical having up to 4 C atoms or by allylthio; a cycloaliphatic radical having 5 to 7 C atoms; a bicycloaliphatic radical having 7 to 14 C atoms; a tricycloaliphatic radical having 7 to 16 C atoms; an alkoxy radical having 1 to 6 C atoms, which can also be substituted by alkoxy having 1 to 6 C atoms; an aryloxy radical having 6 to 10 C atoms; an alkoxycarbonyl radical having a total of 2 to 7 C atoms; an aryl radical having 6 to 10 C atoms; an aryl radical having 6 to 10 C atoms which is mono-, di- or trisubstituted by

1 to 3 halogen atoms and/or 1 to 3 alkyl radicals having 1 to 3 C atoms and/or 1 to 3 alkoxy radicals having 1 to 3 C atoms and/or 1 or 2 nitro groups and/or 1 or 2 hydroxyl groups and/or 1 or 2 alkylcarbonyloxy radicals having 1 to 4 C atoms in the alkyl portion; or imidazolyl and

$R^4$, $R^5$, $R^6$ denote hydrogen or alkyl having 1 to 6 C atoms.

2. Substituted 3-aminosydnonimines according to Claim 1, characterized in that $R^1$ denotes alkyl having 1 to 6 C atoms, hydroxyalkyl having 2 to 6 C atoms, cyclohexyl, hydroxycyclohexyl or adamantyl.

3. Substituted 3-aminosydnonimines according to Claim 1 and/or 2, characterized in that $R^2$ denotes hydrogen, acetyl, propionyl, i-propylcarbonyl, tert.-butylcarbonyl, cyclohexylcarbonyl, benzoyl, 4-chlorobenzoyl, methoxycarbonyl, ethoxycarbonyl, i-propoxycarbonyl, butoxycarbonyl or allylthiomethylcarbonyl.

4. 3-Cyclohexylamino-sydnonimine and its pharmacologically acceptable acid addition salts, in particular its hydrochloride.

5. 3-(Adamant-1-yl)-amino-sydnonimine and its pharmacologically acceptable acid addition salts, in particular its hydrochloride.

6. 3-(2-Hydroxycyclohexyl)-amino-sydnonimine and its pharmacologically acceptable acid addition salts, in particular its hydrochloride.

7. Process for the preparation of the compounds of the formula I mentioned in one or more of Claims 1 to 3 or of the compounds mentioned in Claims 4 to 6, characterized in that

a) a compound of the general formula II

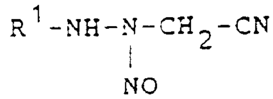

(II)

wherein $R^1$ has the meaning already given, is cyclized to give a compound of the general formula Ia

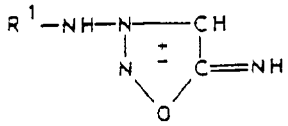

(Ia)

and in that this compound or an acid addition salt thereof, in the case where a compound of the formula I where $R^2$ = -$COR^3$ is to be prepared, is acylated with an acylating agent which introduces the radical -$COR^3$, or, in the case where a compound of the formula I where $R^2$ = -NO is to be prepared, is nitrosated, and if appropriate the compound thus obtained is converted into a pharmacologically acceptable acid addition salt, or in that

b) the tert.-butyl radical is split off from a 3-aminosydnonimine of the formula III

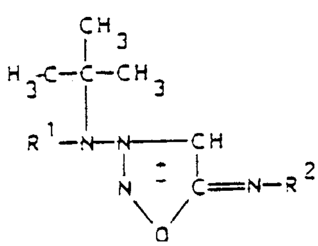

(III)

wherein $R^1$ and $R^2$ have the meanings already given, which can also be in the form of an acid addition salt, and is replaced by hydrogen, a compound of the formula Ia initially being formed, and in that, in the case where a compound of the formula I where $R^2$ = -$COR^3$ is prepared, the compound of the formula Ia or an acid addition salt thereof is acylated with an acylating agent which introduces the radical -$COR^3$, or in the case where a compound of the formula I where $R^2$ = -NO is to be prepared, is nitrosated, and if appropriate the resulting compound is converted into a pharmacologically acceptable acid addition salt.

8. Process according to Claim 7, characterized in that the cyclization or splitting off of the tert.-butyl radical is carried out in a solvent, dispersing agent or diluent at temperatures of 0 to 40°C, preferably 0 to 20°C, with the aid of an acid which establishes a pH of 3 or less in aqueous solution.

9. Substituted 3-aminosydnonimines of claims 1 to 6 or their pharmacologically acceptable acid addition

salts as pharmacological active compounds for use in combating and preventing cardiovascular diseases.

10. Pharmaceutical preparation, characterized in that it contains a compound of Claims 1 to 6 or a pharmacologically acceptable acid addition salt thereof as the active compound, together with pharmaceutically acceptable excipients and additives and if appropriate also one or more other pharmacological active compounds.

11. Substituted 3-aminosydnonimines of the general formula III

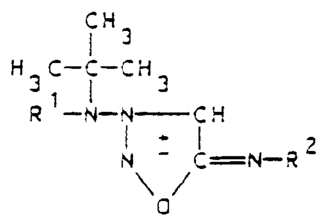

(III)

and their acid addition salts, wherein

$R^1$ denotes alkyl having 1 to 8 C atoms, cycloalkyl having 5 to 8 C atoms, it also being possible for the cycloalkyl or an alkyl radical having 4 to 8 C atoms to be substituted by $-OR^4$ or $-N(R^5, R^6)$, or adamantyl, $R^2$ denotes hydrogen, -NO or the radical $-COR^3$,

$R^3$ denotes an aliphatic radical having 1 to 6 C atoms, which can also be substituted by alkoxy having 1 to 6 C atoms or by an alkyl thio radical having up to 4 C atoms or by allylthio; a cycloaliphatic radical having 5 to 7 C atoms; a bicycloaliphatic radical having 7 to 14 C atoms; a tricycloaliphatic radical having 7 to 16 C atoms; an alkoxy radical having 1 to 6 C atoms, which can also be substituted by alkoxy having 1 to 6 C atoms; an aryloxy radical having 6 to 10 C atoms; an alkoxycarbonyl radical having a total of 2 to 7 C atoms; an aryl radical having 6 to 10 C atoms; an aryl radical having 6 to 10 C atoms which is mono-, di- or trisubstituted by 1 to 3 halogen atoms and/or 1 to 3 alkyl radicals having 1 to 3 C atoms and/or 1 to 3 alkoxy radicals having 1 to 3 C atoms and/or 1 or 2 nitro groups and/or 1 or 2 hydroxyl groups and/or 1 or 2 alkylcarbonyloxy radicals having 1 to 4 C atoms in the alkyl portion; or imidazolyl and

$R^4$, $R^5$, $R^6$ denote hydrogen or alkyl having 1 to 6 C atoms.

**Claims for the following Contracting States : ES, GR**

1. Process for preparing pharmacologically active substituted 3-aminosydnonimines of the general formula I

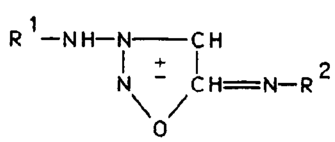

(I)

and their pharmacologically acceptable acid addition salts, wherein

$R^1$ denotes alkyl having 1 to 8 C atoms, cycloalkyl having 5 to 8 C atoms, it also being possible for the alkyl or cycloalkyl radical to be substituted by $-OR^4$ or $-N(R^5, R^6)$, or adamantyl,

$R^2$ denotes hydrogen, -NO or the radical $-COR^3$,

$R^3$ denotes an aliphatic radical having 1 to 6 C atoms, which can also be substituted by alkoxy having 1 to 6 C atoms or by an alkyl thio radical having up to 4 C atoms or by allylthio; a cycloaliphatic radical having 5 to 7 C atoms; a bicycloaliphatic radical having 7 to 14 C atoms; a tricycloaliphatic radical having 7 to 16 C atoms; an alkoxy radical having 1 to 6 C atoms, which can also be substituted by alkoxy having 1 to 6 C atoms; an aryloxy radical having 6 to 10 C atoms; an alkoxycarbonyl radical having a total of 2 to 7 C atoms; an aryl radical having 6 to 10 C atoms; an aryl radical having 6 to 10 C atoms which is mono-, di- or trisubstituted by 1 to 3 halogen atoms and/or 1 to 3 alkyl radicals having 1 to 3 C atoms and/or 1 to 3 alkoxy radicals having 1 to 3 C atoms and/or 1 or 2 nitro groups and/or 1 or 2 hydroxyl groups and/or 1 or 2 alkylcarbonyloxy radicals having 1 to 4 C atoms in the alkyl portion; or imidazolyl and

$R^4$, $R^5$, $R^6$ denote hydrogen or alkyl having 1 to 6 C atoms, characterized in that

a) a compound of the general formula II

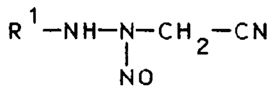

(II)

wherein $R^1$ has the meaning already given, is cyclized to give a compound of the general formula Ia

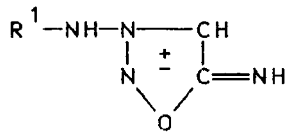

(Ia)

and in that this compound or an acid addition salt thereof, in the case where a compound of the formula I where $R^2$ = $-COR^3$ is to be prepared, is acylated with an acylating agent which introduces the radical $-COR^3$, or, in the case where a compound of the formula I where $R^2$ = -NO is to be prepared, is nitrosated, and if appropriate the compound thus obtained is converted into a pharmacologically acceptable acid addition salt, or in that

b) the tert.-butyl radical is split off from a 3-aminosydnonimine of the formula III

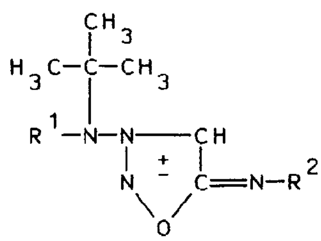

(III)

wherein $R^1$ and $R^2$ have the meanings already given, which can also be in the form of an acid addition salt, and is replaced by hydrogen, a compound of the formula Ia initially being formed, and in that, in the case where a compound of the formula I where $R^2$ = $-COR^3$ is prepared, the compound of the formula Ia or an acid addition salt thereof is acylated with an acylating agent which introduces the radical $-COR^3$, or in the case where a compound of the formula I where $R^2$ = -NO is to be prepared, is nitrosated, and if appropriate the resulting compound is converted into a pharmacologically acceptable acid addition salt.

2. Process according to Claim 1, characterized in that the cyclization or splitting off of the tert.-butyl radical is carried out in a solvent, dispersing agent or diluent at temperatures of 0 to 40°C, preferably 0 to 20°C, with the aid of an acid which establishes a pH of 3 or less in aqueous solution.

3. Process according to Claim 1 or 2, characterized in that the starting products are selected such that a compound of formula I is formed wherein $R^1$ denotes alkyl having 1 to 6 C atoms, hydroxyalkyl having 2 to 6 C atoms, cyclohexyl, hydroxycyclohexyl or adamantyl.

4. Process according to one or several of Claims 1 to 3, characterized in that the starting products are selected such that a compound of formula I is formed wherein $R^2$ denotes hydrogen, acetyl, propionyl, i-propylcarbonyl, tert.-butylcarbonyl, cyclohexylcarbonyl, benzoyl, 4-chlorobenzoyl, methoxycarbonyl, ethoxycarbonyl, i-propoxycarbonyl, butoxycarbonyl or allylthiomethylcarbonyl.

5. Process according to one or several of Claims 1 to 4, characterized in that the starting products are selected such that the compound 3-cyclohexylamino-sydnonimine or a pharmacologically acceptable acid addition salt thereof, in particular its hydrochloride, is formed.

6. Process according to one or several of Claims 1 to 4, characterized in that the starting products are selected such that the compound 3-(adamant-1-yl)-aminosydnonimine or a pharmacologically acceptable acid addition salt thereof, in particular its hydrochloride, is formed.

7. Process according to one or several of Claims 1 to 4, characterized in that the starting products are selected such that the compound 3-(2-hydroxycyclohexyl)amino-sydnonimine or a pharmacologically acceptable acid addition salt thereof, in particular its hydrochloride, is formed.

8. Application of the substituted 3-aminosydnonimine prepared according to the process of one or several of claims 1 to 7 or one of its pharmacologically acceptable acid addition salts as pharmacological active compounds for use in combating and preventing cardiovascular diseases.

9. Process for preparing a pharmaceutical preparation, characterized in that a substituted 3-aminosydnonimine prepared according to the process of one or several of Claims 1 to 7 or a pharmacologically acceptable acid addition salt thereof is converted, together with pharmaceutically acceptable excipients or additives and, if appropriate, one or several other pharmacological active substances, in a manner known per se, into a pharmaceutical preparation.

10. Process for preparing substituted 3-aminosydnonimines of the general formula III

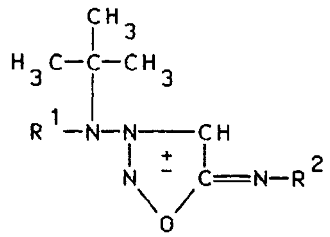

(III)

and their acid additon salts, wherein

$R^1$ denotes alkyl having 1 to 8 C atoms, cycloalkyl having 5 to 8 C atoms, it also being possible for the cycloalkyl radical or an alkyl radical having 4 to 8 C atoms to be substituted by $-OR^4$ or $-N(R^5, R^6)$, or adamantyl,

$R^2$ denotes hydrogen, -NO or the radical $-COR^3$,

$R^3$ denotes an aliphatic radical having 1 to 6 C atoms, which can also be substituted by alkoxy having 1 to 6 C atoms or by an alkyl thio radical having 1 to 4 C atoms or by allylthio; a cycloaliphatic radical having 5 to 7 C atoms; a bicycloaliphatic radical having 7 to 14 C atoms; a tricycloaliphatic radical having 7 to 16 C atoms; an alkoxy radical having 1 to 6 C atoms, which an also be substituted by alkoxy having 1 to 6 C atoms; an aryloxy radical having 6 to 10 C atoms; an alkoxycarbonyl radical having a total of 2 to 7 C atoms; an aryl radical having 6 to 10 C atoms; an aryl radical having 6 to 10 C atoms which is mono-, di- or trisubstituted by 1 to 3 halogen atoms and/or 1 to 3 alkyl radicals having 1 to 3 C atoms and/or 1 to 3 alkoxy radicals having 1 to 3 C atoms and/or 1 or 2 nitro groups and/or 1 or 2 hydroxyl groups and/or 1 or 2 alkylcarbonyloxy radicals having 1 to 4 C atoms in the alkyl portion; or imidazolyl and

$R^4$, $R^5$, $R^6$ denote hydrogen or alkyl having 1 to 6 C atoms, characterized in that a compound of the general formula VII

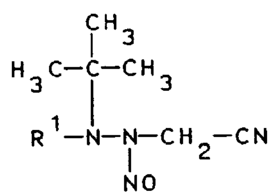

(VII)

is cyclized.

## Revendications

### Revendications pour les Etats contractants suivants : DE, GB, FR, IT, LI, NL, SE, LI, CH, BE, AT

1. 3-aminosydnonimines substituées, pharmacologiquement actives, répondant à la formule générale I

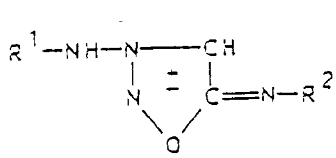

(I)

et leurs sels d'addition d'acides pharmacologiquement acceptables, Composés dans lesquels :

$R^1$ représente un groupe alkyle ayant 1 à 8 atomes de carbone, cycloalkyle ayant 1 à 8 atomes de carbone, cycloalkyle ayant 5 à 8 atomes de carbone, le reste alkyle ou cycloalkyle pouvant également porter un reste $OR^4$ ou $-N(R^5, R^6)$ comme substituant, ou $R^1$ représente un reste adamantyle,

$R^2$ représente un atome d'hydrogène, NO ou le reste $-COR^3$,

$R^3$ représente un reste aliphatique comportant 1 à 6 atomes de carbone, (qui peut porter également comme substituant un groupe alcoxy ayant 1 à 6 atomes de carbone ou un reste alkylthio ayant 1 à 4 atomes de carbone ou un reste allylthio); un reste cycloaliphatique ayant 5 à 7 atomes de carbone ; un reste bicycloaliphatique ayant 7 à 14 atomes de carbone; un reste tricycloaliphatique comportant 7 à 16 atomes de carbone, un reste alcoxy comportant 1 à 6 atomes de carbone (qui peut également porter comme substituant un reste alcoxy ayant 1 à 6 atomes de carbone); un reste aryloxy ayant 6 à 10 atomes de carbone ; un reste alcoxycarbonyle ayant au total 2 à 7 atomes de carbone; un reste aryle ayant 6 à 10 atomes de carbone; un reste aryle ayant 6 à 10 atomes de carbone, monosubstitué, disubstitué ou trisubstitué par 1 à 3 atomes d'halogène et/ou par 1 3 restes alkyles ayant 1 à 3 atomes de carbone et/ou par 1 à 3 restes alcoxy ayant 1 à 3 atomes de carbone et/ou par un ou deux groupes nitro et/ou par un ou deux groupes hydroxy et /ou par un ou deux restes alkylcarbonyloxy ayant 1 à 4 atomes de carbone dans la partie alkyle; un reste imidazolyle;

$R^4$, $R^5$, et $R^6$ représentent chacun un atome d'hydrogène ou un reste alkyle ayant 1 à 6 atomes de carbone.

2. 3-aminosydnonimines substituées selon la revendication 1, caractérisées en ce que $R^1$ représente un reste alkyle ayant 1 à 6 atomes de carbone, hydroxyalkyle ayant 2 à 6 atomes de carbone, cyclohexyle , hydroxycyclohexyle ou adamantyle.

3. 3-aminosydnonimines substituées selon la revendication 1 et/ou 2, caractérisées en ce que $R^2$ représente un atome d'hydrogène, un reste acétyle, propionyle, isopropylcarbonyle, tertiobutylcarbonyle,tertiobutylcarbonyle,cyclohexylcarbonyle, benzoyle, 4-chlorobenzoyle, méthoxycarbonyle, éthoxycarbonyle, isopropoxycarbonyle, butoxycarbonyle, butoxycarbonyle ou allylthiométhylcarbonyle.

4. 3-cyclohexylamino-sydnonimine et ses sels d'addition d'acides pharmacologiquement acceptables, en particulier son chlorhydrate.

5. 3-(adamant-1 yl)-amino-sydnonimine et ses sels d'addition d'acides pharmacologiquemest acceptables, en particulier son chlorhydrate.

6. 3-2-hydroxycyclohexyl)-amino-sydnonimine et ses sels d'addition d'acides pharmacologiquement acceptables, en particulier son chlorhydrate.

7. Procédé pour préparer les composés de formule I, indiqués dans une ou plusieurs des revendications 1 à 3, ou les composés indiqués dans les revendications 4 à 6, caractérisé en ce que :

a) On cyclise un composé de formule générale II

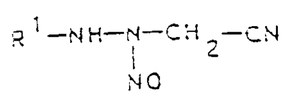

( II )

(dans laquelle $R^1$ a le sens déjà indiqué) pour obtenir un composé de formule générale Ia

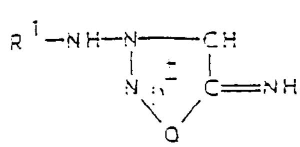

( Ia )

et l'on soumet ce composé, ou un sel d'addition d'acide de ce composé au cas où il faut préparer un composé de formule I dans laquelle $R^2 = -COR^3$, à une acylation à l'aide d'un agent d'acylation qui introduit le reste $-COR^3$ ou, au cas ou l'on doit préparer un composé de formule I dans laquelle $R^2$-NO, à une nitrosation et l'on transforme le composé ainsi obtenu éventuellement en un sel d'addition d'acide pharmacologiquement acceptables, ou bien en ce que

b) on enlève le reste tertiobutyle d'une 3-aminosydnonimine de formule III

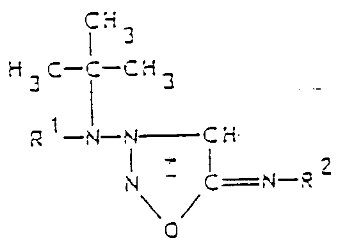

( I I I )

(dans laquelle $R^1$ et $R^2$ ont les sens déjà indiqués) et qui peut également se présenter sous forme d'un sel d'addition d'acide, et l'on remplace ce reste tertiobutyle par un atome d'hydrogène, ce qui donne tout d'abord un composé de formule Ia et, au cas où l'on prépare un composé de formule I dans laquelle $R^2$ représente $COR^3$, on soumet le composé de formule Ia, ou un sel d'addition d'acide de celui-ci, à une acylation à l'aide d'un agent d'acylation qui introduit le reste $-COR^3$, ou, au cas où l'on doit préparer un composé de formule I dans laquelle $R^2$ représente -NO, on soumet à une nitrosation et l'on transforme éventuellement le composé ainsi obtenu en un sel d'addition d'acide pharmacologiquement acceptable.

8. Procédé selon la revendication 7, caractérisé en ce qu'on conduit la cyclisation ou la séparation du reste tertiobutyle en opérant dans un solvant, un agent de dispersion ou un diluant à des températures de 0 à 40° C, avantageusement 0 à 20°C , à l'aide d'un acide qui donne en solution aqueuse un pH égal ou inférieur à 3.

9. 3-aminosydnonimines substituées, selon les revendications 1 à 6, ou leurs sels d'addition d'acides pharmacologiquement acceptables à titre de substances pharmacologiquement actives à utiliser pour combattre et prévenir des maladies cardio-vasculaires.

10. Préparation pharmaceutique, caractérisée en ce qu'elle contient un composé selon une des revendications 1 à 6 ou un sel d'addition d'acide pharmacologiquement acceptable de ce composé, à titre de substance active, avec des excipients ou véhicules et des additifs pharmaceutiquement acceptables, et éventuellement encore une ou plusieurs autres substances pharmacologiquement actives.

11. 3-aminosydnonimines substituées , de formule générale III :

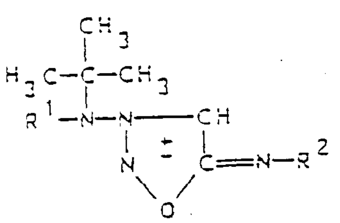

( I I I )

et leurs sels d'addition d'acide, formule dans laquelle :

$R^1$ représente un reste alkyle ayant 1 à 8 atomes de carbone, cycloalkyle ayant 5 à 8 atomes de carbone, le reste cycloalkyle ou un reste alkyle ayant 4 à 6 atomes de carbone pouvant aussi être substitué par $-OR^4$ ou par $-N(R^5, R^6)$ ou bien $R^1$ représente un reste adamantyle,

$R^2$ représente un atome d'hydrogène, -NO ou le reste $-COR^3$,

$R^3$ représente un reste aliphatique ayant 1 à 6 atomes de carbone, (qui peut également être substitué par un reste alcoxy ayant 1 à 6 atomes de carbone ou par un reste alkylthio ayant 1 à 4 atomes de carbone, ou par un reste allylthio); un reste cycloaliphatique ayant 5 à 7 atomes de carbone ; un reste bicycloaliphatique ayant 7 à 14 atomes de carbone; un reste tricycloaliphatique ayant 7 à 16 atomes de carbone, un reste alcoxy ayant 1 à 6 atomes de carbone (qui peut également être substitué par un reste alcoxy ayant 1 à 6 atomes de carbone); un reste aryloxy ayant 6 à 10 atomes de carbone; un reste alcoxycarbonyle ayant au total 2 à 7 atomes de carbone; un reste aryle ayant 6 à 10 atomes de carbone; un reste aryle ayant 6 à 10 atomes de carbone, monosubstitués, disubstitués, ou trisubstitués par 1 à 3 atomes d'halogène et/ou par un ou trois restes alkyle ayant 1 à 3 atomes de carbone et/ou par un à trois restes alcoxy ayant 1 à 3 atomes de carbone et/ou par un ou deux groupes nitro et/ou par un ou deux groupes hydroxy et/ou par un ou deux restes alkylcarbonyloxy ayant 1 à 4 atomes dans la partie alkyle; un reste imidazolyle;

$R^4$ , $R^5$, et $R^6$ représentent chacun un atome d'hydrogène ou un reste alkyle ayant 1 à 6 atomes de carbone.

**Revendications pour les Etats contractants suivants : GR, ES**

1. Procédé pour préparer des 3-aminosydnonimines substituées, à action pharmacologique, répondant à la formule générale (I):

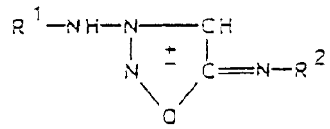

(I)

et leurs sels d'addition d'acides pharmacologiquement acceptables, composé dans lesquels :

$R^1$ représente un groupe alkyle ayant 1 à 8 atomes de carbone, cycloalkyle ayant 1 à 8 atomes de carbone, cycloalkyle ayant 5 à 8 atomes de carbone, le reste alkyle ou cycloalkyle pouvant également porter un reste $OR^4$ ou $-N(R^5, R^6)$ comme substituant, ou $R^1$ représente un reste adamantyle,

$R^2$ représente un atome d'hydrogène, -NO ou le reste $-COR^3$,

$R^3$ représente un reste aliphatique comportant 1 à 6 atomes de carbone, (qui peut porter également comme substituant un groupe alcoxy ayant 1 à 6 atomes de carbone ou un reste alkylthio ayant 1 à 4 atomes de carbone ou un reste allylthio); un reste cycloaliphatique ayant 5 à 7 atomes de carbone ; un reste bicycloaliphatique ayant 7 à 14 atomes de carbone; un reste tricycloaliphatique comportant 7 à 16 atomes de carbone, un reste alcoxy comportant 1 à 6 atomes de carbone (qui peut également porter comme substituant un reste alcoxy ayant 1 à 6 atomes de carbone); un reste aryloxy ayant 6 à 10 atomes de carbone ; un reste alcoxycarbonyle ayant au total 2 à 7 atomes de carbone; un reste aryle ayant 6 à 10 atomes de carbone; un reste aryle ayant 6 à 10 atomes de carbone, monosubstitué , disubstitué ou trisubstitué par 1 à 3 atomes d'halogène et/ou par 1 à 3 restes alkyles ayant 1 à 3 atomes de carbone et/ou par 1 à 3 restes alcoxy ayant 1 à 3 atomes de carbone et/ou par un ou deux groupes nitro et/ou par un ou deux groupes hydroxy et /ou par un ou deux restes alkylcarbonyloxy ayant 1 à 4 atomes de carbone dans la partie alkyle; un reste aryle mono,di ou trisubstitué ayant 6 à 10 atomes de carbone, un reste imidazolyle;

$R^4$, $R^5$, et $R^6$ représentent chacun un atome d'hydrogène, ou un reste alkyle ayant 1 à 6 atomes de carbone. procédé caractérisé en ce que :

a) On cyclise un composé de formule générale II

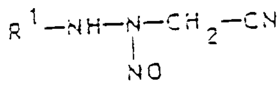

(II)

(dans laquelle $R^1$ a le sens déjà indiqué) pour obtenir un composé de formule générale Ia

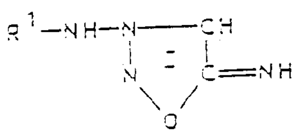

(Ia)

et l'on soumet ce composé, ou un sel d'addition d'acide de ce composé au cas où il faut préparer un composé de formule I dans laquelle $R^2 = -COR^3$, à une acylation à l'aide d'un agent d'acylation qui introduit le reste $COR^3$ ou, au cas ou l'on doit préparer un composé de formule I dans laquelle $R^2$-NO, à une nitrosation et l'on transforme le composé ainsi obtenu éventuellement en un sel d'addition d'acide pharmacologiquement acceptable, ou bien en ce que

b) on enlève le reste tertiobutyle d'une 3-aminosydnonimine de formule III

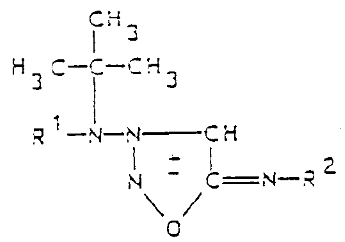

( I I I )

(dans laquelle $R^1$ et $R^2$ ont les sens déjà indiqués) et qui peut également se présenter sous forme d'un sel d'addition d'acide, et l'on remplace ce reste tertiobutyle par un atome d'hydrogène, ce qui donne tout d'abord un composé de formule Ia et, au cas où l'on prépare un composé de formule I dans laquelle $R^2$ représente $-COR^3$ , on soumet le composé de formule Ia ou un sel d'addition d'acide de celui-ci à une acylation à l'aide d'un agent d' acylation qui introduit le reste $-COR^3$, ou, au cas où l'on doit préparer un composé de formule I dans laquelle $R^2$ représente -NO, on soumet à une nitrosation et l'on transforme éventuellement le composé ainsi obtenu en un sel d'addition d'acide pharmacologiquement acceptable .

2. Procédé selon la revendication 1, caractérisée en ce qu'on conduit la cyclisation ou la séparation du reste tertiobutyle en opérant dans un solvant, un dispersant ou un diluant à des températures de 0 à 40° C, avantageusement de 0 à 20°C, à l'aide d'un acide qui donne en solution aqueuse un pH égal ou inférieur à 3.

3. Procédé selon la revendication 1 et/ou 2, caractérisé en ce qu'on choisit les produits de départ de façon qu'il en résulte un composé de formule I, dans laquelle $R^1$ représente un reste alkyle ayant 1 à 6 atomes de carbone, hydroxyalkyle ayant 2 à 6 atomes de carbone, cyclohexyle, hydroxycyclohexyle ou adamantyle.

4. Procédé selon une ou plusieurs des revendications 1 à 3, caractérisé en ce qu'on choisit les produits de départ de façon qu'il en résulte un composé de formule I , dans laquelle : $R^2$ représente un atome d'hydrogène, un reste acétyle, propionyle, isopropylcarbonyle, tertiobutylcarbonyle,tertiobutylcarbonyle,cyclohexylcarbonyle, benzoyle, 4-chlorobenzoyle, méthoxycarbonyle, éthoxycarbonyle, isopropoxycarbonyle, butoxycarbonylecarbonyle, ou allylthiométhylcarbonyle.

5. Procédé selon une ou plusieurs des revendications 1 à 4, caractérisé' en ce qu'on choisit les produits de départ de façon à ce qu'il en résulte le composé 3-cyclohexylamino-sydnonimine ou un sel d'addition d'acide phrmacologiquement acceptable de ce composé, en particulier son chlorhydrate.

6. Procédé selon une ou plusieurs des revendications 1 à 4 caractérisé, en ce qu'on choisit les produits de départ de façon qu'il en résulte le composé 3-(adamant-1-yl) -amino-sydnonimine ou un sel d'addition d'acide pharmacologiquement acceptable de ce composé, en particulier son chlorhydrate.

7. Procédé selon une ou plusieurs des revendications 1 à 4 caractérisé, en ce qu'on choisit les produits de départ de façon qu'il en résulte le composé 3-(2-hydroxycyclohexyl) - amino-sydnonimine ou un sel d'addition d'acide pharmacologiquement acceptable dérivant de ce composé, en particulier son chlorhydrate.

8. Application de la 3-amino-sydnonimine substituée, préparée selon une ou plusieurs des revendications 1 à 7, ou d'un de ses sels d'addition d'acide pharmacologiquement acceptable, comme une substance pharmacologiquement active, dans la lutte contre les maladies cardio-vasculaires et pour la prévention de telles maladies.

9. Procédé pour confectionner une préparation pharmaceutique, caractérisé en ce qu'on introduit une 3-amino-sydnonimine substituée, préparée selon le procédé d'une ou plusieurs des revendications 1 à 7, ou un sel d'addition d'acide pharmacologiquement acceptable de ce composé, comme substance active avec des véhicules , excipients et additifs pharmaceutiquement acceptables et éventuellement encore avec une ou plusieurs autres substances pharmacologiquement actives, de façon connue, dans une préparation pharmaceutique.

10. Procédé pour préparer des 3-aminosydnonimines substituées , de formule générale III

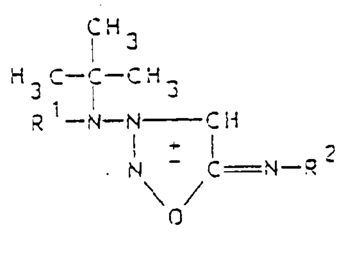

( I I I )

et leurs sels d'addition d'acide, formule dans laquelle

$R^1$ représente un reste alkyle ayant 1 à 8 atomes de carbone, cycloalkyle ayant 1 à 5 atomes de carbone, le reste cycloalkyle ou un reste alkyle ayant 4 à 6 atomes de carbone pouvant aussi être substitué par $-OR^4$ ou par $-N(R^5, R^6)$ ou bien $R^1$ représente un reste adamantyle,

$R^2$ représente un atome d'hydrogène, -NO ou le reste $-COR^3$,

$R^3$ représente un reste aliphatique ayant 1 à 6 atomes de carbone, (qui peut également être substitué par un reste alcoxy ayant 1 à 6 atomes de carbone ou par un reste alkylthio ayant 1 à 4 atomes de carbone, ou par un reste allylthio); un reste cycloaliphatique ayant 5 à 7 atomes de carbone ; un reste bicycloaliphatique ayant 7 à 14 atomes de carbone; un reste tricycloaliphatique ayant 7 à 16 atomes de carbone, un reste alcoxy ayant 1 à 6 atomes de carbone (qui peut également être substitué par un reste alcoxy ayant 1 à 6 atomes de carbone); un reste aryloxy ayant 6 à 10 atomes de carbone; un reste alcoxycarbonyle ayant au total 2 à 7 atomes de carbone; un reste aryle ayant 6 à 10 atomes de carbone; un reste aryle ayant 6 à 10 atomes de carbone, monosubstitués, disubstitués, ou trisubstitués par 1 à 3 atomes d'halogène et/ou par un ou trois restes alkyl ayant 1 à 3 atomes de carbone et/ou par un à trois restes alcoxy ayant 1 à 3 atomes de carbone et/ou par un ou deux groupes nitro et/ou par un ou deux groupes hydroxy et/ou par un ou deux restes alkylcarbonyloxy ayant 1 à 4 atomes dans la partie alkyle; un reste imidazolyle;

$R^4$ , $R^5$, et $R^6$ représentent chacun un atome d'hydrogène ou un reste alkyle ayant 1 à 6 atomes de carbone, procédé caractérisé en ce qu'on cyclise un composé de formule générale VII

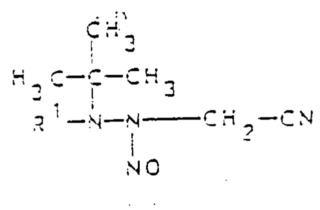

(VII)